# EUROPÄISCHE PATENTSCHRIFT

(11) **EP 2 470 518 B1**
(45) Veröffentlichungstag und Bekanntmachung des Hinweises auf die Patenterteilung: **16.04.2014**
(21) Anmeldenummer: 10742837.7
(22) Anmeldetag: 17.08.2010
(51) Int. Cl.: C07D 307/68, C08K 5/12, C08K 5/1535

(54) **ESTERDERIVATE DER 2,5-FURANDICARBONSÄURE UND IHRE VERWENDUNG ALS WEICHMACHER**
2,5-FURAN DICARBOXYLATE DERIVATIVES, AND USE THEREOF AS PLASTICIZERS
DÉRIVÉS ESTER D'ACIDE 2,5-FURANE-DICARBOXYLIQUE ET LEUR UTILISATION COMME PLASTIFIANT

(30) Priorität: 28.08.2009 DE 102009028975
(43) Veröffentlichungstag der Anmeldung: 04.07.2012
(73) Patentinhaber: Evonik Degussa GmbH, 45128 Essen (DE)
(72) Erfinder: GRASS, Michael, 45721 Haltern am See (DE); BECKER, Hinnerk Gordon, 45257 Essen (DE)
(86) Internationale Anmeldenummer: PCT/EP2010/061941
(87) Internationale Veröffentlichungsnummer: WO 2011/023590

(56) Entgegenhaltungen:
- SANDERSON R D ET AL: "SYNTHESIS AND EVALUATION OF DIALKYL FURAN-2,5-DICARBOXYLATES AS PLASTICIZERS FOR PVC" JOURNAL OF APPLIED POLYMER SCIENCE, JOHN WILEY AND SONS INC. NEW YORK, US LNKD- DOI:10.1002/APP.1994.070531308, Bd. 53, Nr. 13, 26. September 1994 (1994-09-26), Seiten 1785-1793, XP000464476 ISSN: 0021-8995 in der Anmeldung erwähnt
- CRESPO, J. E. ET AL.: "Substitution of Di(2-ethylhexyl) Phthalate by Di(isononyl)Cyclohexane-1,2-Dicarboxylate as a Plasticizer forIndustrial Vinyl Plastisol Formulations" JOURNAL OF APPLIED POLYMER SCIENCE,, Bd. 104, 2007, Seiten 1215-1220, XP002604948 DOI: 10.1002/app.25760

## Beschreibung

Die vorliegende Erfindung betrifft ein Gemisch aus Estern der 2,5-Furandicarbonsäure (FDCA) mit isomeren C9- Alkoholen, insbesondere Gemischen aus linearen und verzweigten Nonanolen. Ebenfalls betrifft die vorliegende Erfindung ein Verfahren zur Herstellung solcher Ester bzw. Gemische und deren Verwendung als Weichmacher für Polymere wie beispielsweise Polyvinylchlorid.

Polyvinylchlorid (PVC) gehört zu den wirtschaftlich bedeutendsten Polymeren. Es findet sowohl als Hart-PVC als auch als Weich-PVC vielfältige Anwendungen.

Zur Erzeugung eines Weich-PVC werden dem PVC Weichmacher zugesetzt, wobei in der überwiegenden Anzahl der Fälle Phthalsäureester, insbesondere Di-2-ethylhexylphthalat (DEHP), Düsononylphthalat (DINP) und Diisodecylphthalat (DIDP) Verwendung finden. Durch bereits bestehende und möglicherweise künftige gesetzliche Regelungen zum eingeschränkten Einsatz von Phthalaten besteht der Bedarf, neue als Weichmacher für PVC und andere Polymere geeignete Ester zu finden, bei denen bevorzugt die gleichen Alkohole wie bisher zum Einsatz kommen können,. Wegen der begrenzten Verfügbarkeit fossiler Rohstoffe sollten insbesondere solche Ester zukünftig gute Marktchancen haben, bei denen zumindest die Säurekomponente auf natürlich vorkommenden Ressourcen wie Zucker, Fetten oder Ölen basiert.

In der Veröffentlichung "Top Value Added Chemicals from Biomass" von T. Werpy und G. Petersen (U.S. Dept. Of Energy (DOE); 08/2004), wird 2,5-Furandicarbonsäure (FDCA) als einer der aussichtsreichsten Plattformchemikalien auf Basis von Zucker angesehen. Wegen der strukturellen Ähnlichkeit mit Terephthalsäure wurden in den letzten Jahren zahlreiche Arbeiten zur Verwendung der 2,5-Furandicarbonsäure oder verschiedener Derivate, überwiegend in Polymeren publiziert. Die Hauptanwendung war in der Mehrzahl der Fälle der teilweise oder vollständige Ersatz von Terephthalsäure oder ihrer Derivate in Polymeren.

Eine sehr umfangreiche Übersicht über FDCA, seine Anwendungen und Möglichkeiten zur Synthese findet sich in der im Internet veröffentlichten Publikation von Jaroslaw Lewkowski, ARKIVOC 2001 (i), Seiten 17-54, ISSN 1424-6376, mit dem Titel "Synthesis, Chemistry and Applications of 5-hydroxymethylfurfural and its derivatives". Den meisten dieser Synthesen gemein ist eine säurekatalysierte Umsetzung von Kohlenhydraten, besonders Glucose oder Fructose, bevorzugt Fructose zum 5-Hydroxymethylfurfural (5-HMF) welches durch verfahrenstechnische Operationen wie beispielsweise Zweiphasen-Fahrweise aus dem Reaktionsmedium abgetrennt werden kann. Entsprechende Ergebnisse wurden beispielsweise von Roman-Leshkov et al. in Science 2006, 312, Seite 1933-1937 und von Zhang in Angewandte Chemie 2008, 120, Seiten 9485-9488 beschrieben.

In einem weiteren Schritt kann 5-HMF dann zu FDCA oxidiert werden, wie z. B. von Christensen in ChemSusChem 2007, 1, S. 75 - 78 zitiert.

Weiterhin ist die Herstellung bestimmter FDCA-Ester auch durch eine direkte Synthese ausgehend von Schleimsäure (Tagouchi in Chemistry Letter Vol. 37, No.1 (2008)) und den entsprechenden Alkoholen beschrieben.

J. E. Crespo et al. Journal of Appl. Pol. Sci., Vol. 104, 2007, S. 1215-1220, offenbart Diisononylcyclohexan-1,2-dicarbonsäureestern.

Die Verwendung von Estern der 2,5-Furandicarbonsäure als Weichmacher für Kunststoffe, insbesondere PVC, PVB, PLA, PHB oder PAMA ist bisher nur selten beschrieben. Die umfangreichste Übersicht in diesem Zusammenhang findet sich in der Veröffentlichung von R.D. Sanderson et al. in Journal of Appl. Pol. Sci. 1994, Vol. 53, S. 1785 bis 1793. Dort werden explizit die entsprechenden Ester auf Basis von n-Butanol, n-Hexanol, 2-Octanol und 2-Ethylhexanol beschrieben. Die Untersuchungen zur Wechselwirkung der Ester mit PVC zeigten, dass diese als Weichmacher für PVC Verwendung finden könnten. Allerdings wurden diese Schlussfolgerungen lediglich aus DMTA-Messungen abgeleitet. Für den Verarbeiter wichtige und aussagekräftigere anwendungstechnische Untersuchungen wurden nicht durchgeführt. Beispielsweise ist hier auch nicht erwähnt, dass der 2-Ethylhexylester der FDCA bei tieferen Temperaturen zur Kristallisation neigt, wie durch DSC-Messungen (Schmelzpunktmaximum bei 12 °C mit Onset bei -2,7 °C) belegt werden kann. Hiermit wird dieser Ester für viele Verarbeiter nur beschränkt einsetzbar sein, da bei tiefen Temperaturen keine Pumpbarkeit mehr gegeben ist.

Hinzu kommt die Einstufung von 2-Ethylhexanol als Gefahrstoff, welche die Einsetzbarkeit, insbesondere in Bereichen mit Haut- und/oder Lebensmittelkontakt einschränkt.

Ausgehend von dem bekannten Stand der Technik bestand daher die Aufgabe, Ester auf Basis 2,5-Furandicarbonsäure bereitzustellen, die als Weichmacher für Kunststoffe wie zum Beispiel PVC, PVB, PLA, PHB oder PAMA Verwendung finden können, bei denen das vorgenannte Problem nicht oder nur in deutlich abgeschwächter Form auftritt, und die das technische Potential haben, die derzeitigen petrochemischen Standardweichmacher zu ersetzen.

Es wurde gefunden, dass Gemische isomerer Nonylester der 2,5-Furandicarbonsäure (Formel I) als Weichmacher für Kunststoffe, insbesondere PVC, PVB, PLA, PHB und PAMA verwendet werden können und dort vorteilhafte Eigenschaften gegenüber den literaturbekannten FDCA-Estern zeigen. Weiterhin zeigen diese Ester gegenüber den entsprechenden Phthalsäureestern ebenfalls anwendungstechnische Vorteile.

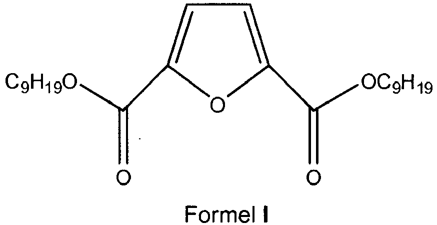

Gegenstand der vorliegenden Erfindung sind Gemische isomerer Nonylester der 2,5-Furandicarbonsäure der Formel I. Weiter sind Gegenstand der vorliegenden Erfindung Zusammensetzungen, enthaltend die Gemische isomerer Nonylester der 2,5-Furandicarbonsäure der Formel I.

Bezüglich der Rohstoffbasis liegt die Besonderheit der vorliegenden Erfindung in der optionalen Nutzung nachwachsender Rohstoffe zur Herstellung der erfindungsgemäßen Furandicarbonsäureester. Dabei versteht man im Sinne der vorliegenden Erfindung unter nachwachsenden Rohstoffen im Unterschied zu petrochemischen Rohstoffen, welche auf fossilen Ressourcen, wie z. B. Erdöl oder Steinkohle basieren, solche Rohstoffe, die auf Basis von Biomasse entstehen bzw. hergestellt werden. Die Begriffe "Biomasse", "biobasiert" oder "basierend auf bzw. hergestellt aus nachwachsenden Rohstoffen" umfassen alle Materialien biologischen Ursprungs, die dem sogenannten "Kohlenstoff-Kurzzeitzyklus" entstammen, somit nicht Bestandteil geologischer Formationen oder Fossilschichten sind. Die Identifizierung und Quantifizierung nachwachsender Rohstoffe erfolgt gemäß ASTM-Methode D6866. Kennzeichnend ist u.a. für nachwachsende Rohstoffe ihr Anteil an dem Kohlenstoffisotop ¹⁴C im Gegensatz zu petrochemischen Rohstoffen.

Ein besonderer ökonomischer und gleichzeitig ökologischer Vorteil der vorliegenden Erfindung liegt in der gleichzeitigen Nutzung von nachwachsenden und petrochemischen Rohstoffen für die Herstellung der erfindungsgemäßen Furandicarbonsäureester, was einerseits eine besonders preiswerte Herstellung und eine breite Anwendbarkeit ermöglicht, andererseits aber auch zu besonders "nachhaltigen" Produkten führt.

Ein weiterer Gegenstand der vorliegenden Erfindung ist auch die Verwendung dieser Gemische in Farben, Tinten oder Lacken, in Plastisolen, Klebstoffen oder Klebstoffkomponenten, in Dichtungsmassen, als Weichmacher in Kunststoffen oder Kunststoffkomponenten, als Lösemittel, als Schmierölkomponente und als Hilfsmittel bei der Metallverarbeitung sowie eine PVC-Zusammensetzung oder ein Plastisol, enthaltend PVC und von 5 bis 250 Massenteilen des erfindungsgemäßen Gemisches pro 100 Massenteile PVC.

Ein weiterer Gegenstand der vorliegenden Erfindung ist ein Verfahren zur Herstellung von Gemischen isomerer Nonylester der 2,5-Furandicarbonsäure, dadurch gekennzeichnet, dass man 2,5-Furandicarbonsäure mit einem Gemisch isomerer Nonanole, im Folgenden Isononanol genannt, optional in Gegenwart eines Katalysators verestert oder 2,5-Furandicarbonsäuredimethylester, mit Isononanol unter Freisetzung von Methanol, optional unter Verwendung eines Katalysators zum Gemisch isomerer Nonylester der 2,5-Furandicarbonsäure umestert.

Weiterhin kann zur Herstellung eines Gemisches isomerer Nonylester auch von Schleimsäure ausgegangen werden, die in Gegenwart von isomeren Nonanolen unter bevorzugt saurer Katalyse im Sinne einer Eintopfreaktion simultan cyclisiert und zum entsprechenden Furandicarbonsäureester umgesetzt wird.

Gegenüber Furandicarbonsäureestern gemäß dem Stand der Technik aber auch gegenüber dem derzeitigen Standardweichmacher Di(isononyl)phthalat (DINP), weisen die erfindungsgemäßen Gemische isomerer Nonylester der FDCA deutlich verbesserte Eigenschaften bei der Verwendung als Weichmacher in Kunststoffen, insbesondere PVC auf.

Gegenüber den aus dem Stand der Technik bekannten FDCA-Estern auf Basis 2-Ethylhexanol weisen die erfindungsgemäßen Di-isononylester eine geringere Flüchtigkeit aus der Folie sowie in Plastisolen einen geringeren Anstieg der Viskosität mit der Zeit und somit eine verbesserte Alterungsbeständigkeit auf. Darüber hinaus zeigt das erfindungsgemäße Estergemisch auf Basis von Isononanol im Gegensatz zum Di-2-ethylhexylester keine Kristallisationsneigung im Bereich bis -20 °C, sondern lediglich einen Glasübergangspunkt bei ca. -80 °. Interessanterweise zeigen die erfindungsgemäßen Diisononylester in für den Anwender zentralen Eigenschaften auch gegenüber dem entsprechenden Phthalat (Diisononylphthalat, DINP) verbesserte Eigenschaften wie zum Beispiel schnellere Gelierung und verbesserte weichmachende Wirkung. Für den Verarbeiter bedeutet dies entweder eine geringere Verarbeitungstemperatur oder bei gleicher Verarbeitungstemperatur einen höheren Produktausstoß pro Zeiteinheit kombiniert mit dem Effekt, für das gleiche Maß an Weichheit / Flexibilität nunmehr weniger Weichmacher zu benötigen als beim entsprechenden Phthalat.

Vorzugsweise sind die erfindungsgemäßen Gemische isomerer Nonylester der 2,5-Furandicarbonsäure so zusammengesetzt, dass das Gemisch mindestens zwei unterschiedliche Ester enthält, die sich in der Konstitution der isomeren C9-Reste unterscheiden, wobei keiner der im Gemisch vorhandenen C9-Reste einen Anteil von mehr als 90 Mol-%, aufweist.

Das erfindungsgemäße Gemisch kann entweder ausschließlich aus den Gemischen der Ester der Formel I bestehen oder neben diesen zumindest ein Polymer und/oder zumindest einen Weichmacher, der kein Diester der Formel I ist, aufweisen. Die Weichmacher können z. B. ausgewählt sein aus Citronensäuretrialkylestern, acylierten Citronensäuretrialkylestern, Glycerinestern, Glykoldibenzoaten, Alkylbenzoaten, Dialkyladipaten, Trialkyltrimellitaten, Dialkylterephthalaten, Dialkylphthalaten oder den Dialkylestern der 1,2-, 1,3- oder 1,4-Cyclohexandicarbonsäuren, wobei die Alkylreste von 4 bis 13, vorzugsweise 5, 6, 7, 8, 9, 10, 11 oder 13 Kohlenstoffatome aufweisen. Die Weichmacher können auch Dianhydrohexitolester, bevorzugt Isosorbiddiester von Carbonsäuren, wie zum Beispiel n- oder iso-Buttersäure, Valeriansäure oder 2-Ethylhexansäure oder Isononansäure sein.

Polymere, welche in dem erfindungsgemäßen Gemisch enthalten sein können, sind z. B. Polyvinylchlorid (PVC), Polyvinylbutyral (PVB), Polymilchsäure (PLA), Polyhydroxybutyral (PHB) und die Polyalkylmethacrylate (PAMA). Besonders bevorzugt ist das Polymer Polyvinylchlorid (PVC).

In bevorzugten Gemischen, die Diester der Formel **I** und Polymere aufweisen, beträgt das Massen-Verhältnis von Polymer/Polymeren zu Diester/-n der Formel **I** vorzugsweise von 30 zu 1 bis 1 zu 2,5 und bevorzugt von 20 zu 1 bis 1 zu 2.

In bevorzugten Gemischen, die Diester der Formel **I** und Weichmacher, die kein Diester der Formel **I** sind, aufweisen, beträgt das Mol-Verhältnis von Weichmachern, insbesondere von Alkylbenzoaten, Dialkyladipaten, Glycerinestern, Citronensäuretrialkylestern, acylierten Citronensäuretrialkylestern, Trialkyltrimellitaten, Glycoldibenzoaten, Dialkylterephthalaten, Dialkylphthalaten, Dialkanoylestern des Isosorbid und/oder den Dialkylestern der 1,2-, 1,3-oder 1,4-Cyclohexandicarbonsäuren, zu Diester/-n der Formel **I** vorzugsweise von 1 zu 15 bis 15 zu 1, bevorzugt von 1 zu 6 bis 6 zu 1.

Die erfindungsgemäßen Gemische von Diestern der Formel **I** bzw. die Diester der Formel **I** selbst, können auf verschiedene Weisen hergestellt werden. Vorzugsweise werden die Gemische von Diestem der Formel **I** bzw. die Diester der Formel **I,** mit dem nachfolgend beschriebenen Verfahren hergestellt.

Das erfindungsgemäße Verfahren zur Herstellung isomerer Nonylester der 2,5-Furandicarbonsäure zeichnet sich dadurch aus, dass 2,5-Furandicarbonsäure oder ein kürzerkettiger Dialkylester dieser Verbindung, besonders der Dimethylester, mit einem Gemisch isomerer Nonanole unter optionaler Verwendung eines Katalysators umgesetzt wird. Weiterhin kann auch das 2,5-Furandicarbonsäuredichlorid, welches durch Umsetzung der FDCA mit Chlorierungsmitteln wie beispielweise Thionylchlorid erhalten werden kann, als Ausgangsstoff zur Herstellung der Diisononylester eingesetzt werden. Geeignete Bedingungen zur Umsetzung von FDCA zum Diisononylester über die Zwischenstufe des Dichlorids finden sich in den Beispielen.

Vorzugsweise wird ein Gemisch isomerer Nonanole eingesetzt, welches mindestens zwei Nonanole der Summenformel C₈H₁₇CH₂OH mit unterschiedlicher Konstitutionsformel aufweist, wobei keiner der im Gemisch vorhandenen Nonylalkohole einen Anteil von bevorzugt mehr als 90 Mol-% aufweist.

Bevorzugt enthalten die im erfindungsgemäßen Verfahren eingesetzten Gemische isomerer Nonanole der Summenformel C₉H₁₀OH, insbesondere der Formel C₈H₁₇CH₂OH, weniger als 10 Mol-%, vorzugsweise weniger als 5 Mol-%, bevorzugt weniger als 1 Mol-% und insbesondere von 0 bis 0,5 Mol-%, bevorzugt weniger als 0,1 Mol-%, insbesondere von 0,0001 bis 0,1 Mol-% und besonders bevorzugt weniger als 0,05 Mol-%, insbesondere von 0,01 bis 0,05 Mol % an 3,5,5-Trimethylhexanol oder anderen dreifach substituierten Nonylalkoholen mit der Summenformel C₈H₁₇CH₂OH, insbesondere solchen mit quartären C-Atomen. Die Anwesenheit dieser Alkohole verschlechtert die anwendungstechnischen Eigenschaften, und reduziert die Geschwindigkeit des biologischen Abbaus des Moleküls.

Es kann außerdem vorteilhaft sein, wenn die zur Herstellung der im erfindungsgemäßen Gemisch enthaltenen Diester der Formel I verwendeten Isononanole der Summenformel C₉H₁₉OH, insbesondere der Formel C₈H₁₇CH₂OH, 1 bis 85 %, insbesondere 1 bis 50 %, bevorzugt 2 bis 20 %, n-Nonanol aufweisen.

Die Isomerenverteilungen der isomeren Alkohole in den Gemischen können mit den üblichen, dem Fachmann geläufigen Messmethoden wie NMR-Spektroskopie, GC- oder GC/MS-Spektroskopie, bevorzugt nach Überführung in die Silyl- oder Methylester, ermittelt werden.

### Synthese der isomeren Nonylalkohole

Prinzipiell können alle technischen Gemische, von Nonanolen mit der Summenformel C₉H₁₉OH, insbesondere solche mit der Formel C₈H₁₇CH₂OH, die zumindest zwei unterschiedliche Konstitutionsisomere aufweisen, eingesetzt werden. Vorzugsweise werden solche Gemische isomerer Nonanole mit der Formel C₈H₁₇CH₂OH eingesetzt, die hinsichtlich des Anteils der verschiedenen Isomeren und/oder des Gehalts an C9-Alkoholen mit quartären C-Atomen in den oben angegebenen Bereichen liegen.

Die im erfindungsgemäßen Verfahren eingesetzten Gemische isomerer Nonanole mit der Summenformel C₉H₁₉OH, insbesondere der Formel C₈H₁₇CH₂OH (nachfolgend Isononanole genannt) können beispielsweise durch Hydroformylierung von Octenen, die wiederum auf unterschiedliche Art erzeugt werden können, und anschließende Hydrierung hergestellt werden.

Als Rohstoff zur Herstellung der Octene können technische C₄-Ströme eingesetzt werden, die zunächst alle isomeren C₄-Olefine neben den gesättigten Butanen und gegebenenfalls Verunreinigungen wie C₃- und C₅-Olefinen und acetylenischen Verbindungen enthalten. Durch Oligomerisierung der in den C₄-Strömen enthaltenen Olefine erhält man vorwiegend isomere Octengemische neben höheren Oligomeren wie C₁₂- und C₁₆-Olefingemischen. Diese Octengemische können, gegebenenfalls nach einer destillativen Abtrennung der C₁₂- und C₁₆-Olefine, zu den entsprechenden Aldehyden hydroformyliert werden und können anschließend zum Alkohol hydriert werden. Die Zusammensetzung, d. h. die Isomerenverteilung der technischen Nonanolgemische ist abhängig vom Ausgangsmaterial und im Wesentlichen abhängig von den Oligomerisierungs- und Hydroformylierungsverfahren.

Als Octen-Gemische können z. B. auch solche eingesetzt werden, die über das sogenannte Polygas-Verfahren erhalten werden, bei dem eine Oligomerisierung von C₃-/C₄-Mischungen an einem festen sauren Katalysator, vorzugsweise an einem festen Phosphorsäure-Katalysator (SPA-Verfahren) durchgeführt wird. Dieses Verfahren wird unter anderem in den Dokumenten US 6,284,938, US 6,080,903, US 6,072,093, US 6,025,533, US 5,990,367, US 5,895,830, US 5,856,604, US 5,847,252 und US 5,081,086 beschrieben. Werden ausschließlich auf diese Weise erhaltene Olefingemische hydroformyliert, so werden in der Regel auch noch Anteile von Octanalen und Decanalen erhalten, so dass hier die mittlere Kettenlänge von 9 Kohlenstoffatomen abweichen kann. Nach der Hydrierung wird also ein isomere Nonanole aufweisendes Gemisch erhalten, welches auch noch isomere Octanole oder Decanole aufweisen kann. Weiterhin können auch Octene aus der Ethylen-Oligomerisierung vorteilhaft verwendet werden.

Besonders bevorzugte, im erfindungsgemäßen Verfahren einsetzbare Gemische isomerer Nonanole sind solche, die erhältlich sind durch Hydroformylierung von isomeren Octenen und anschließende Hydrierung der entstehenden Aldehyde, wobei das Gemisch isomerer Octene durch Inkontaktbringen eines Butene aufweisenden Kohlenwasserstoffgemisches, welches einen Anteil an Isobuten von vorzugsweise kleiner 20 Massen-%, bevorzugt kleiner 10 Massen-%, besonders bevorzugt kleiner 5 Massen-%, ganz besonders bevorzugt kleiner 3 Massen-%, insbesondere bevorzugt kleiner 1 Massen-%, vorzugsweise zwischen 0,01 und 1 Massen-% und besonders bevorzugt zwischen 0,05 und 0,5 Massen-% bezogen auf die Butene aufweist, mit einem Oligomerisierungskatalysator, insbesondere mit einem Nickeloxid enthaltenden Katalysator, erhalten wird. Die Herstellung von isomeren Octenen durch Oligomerisierung von im wesentlichem linearen Butenen an Nickelträgerkatalysatoren ist z. B. als OCTOL-Prozess bekannt, der z. B. in EP 0 395 857 oder EP 1 029 839 beschrieben wird. In Varianten zum OCTOL-Prozess werden z. B. Ti oder Zr aufweisende Katalysatoren eingesetzt. Solche alternativen Varianten und insbesondere die Katalysatoren werden z. B. in EP 1 171 413 beschrieben. Wie bereits oben beschrieben, können die so erhaltenen Octene von den höheren Olefinen, also den C₁₂-, C₁₆-, C₂₀- etc. Olefinen z. B. destillativ abgetrennt werden.

Die z. B. wie oben beschrieben hergestellten Octene oder Gemische isomerer Octene, werden anschließend einer Hydroformylierung zugeführt. Die Hydroformylierung kann in Gegenwart von modifizierten oder unmodifizierten Kobalt- oder Rhodiumkatalysatoren erfolgen. Vorzugsweise erfolgt die Hydroformylierung in Gegenwart von unmodifizierten Kobaltverbindungen. Geeignete Hydroformylierungs-Verfahren sind z. B. aus EP 0 850 905 und EP 1 172 349 bekannt. In der Regel entsteht auf diese Weise ein Gemisch aus im Wesentlichen isomeren Nonanalen, eventuell noch nicht umgesetzten Octenen, sowie den entsprechenden, durch Hydrierung (Folgereaktion) entstehenden Gemischen aus isomeren Nonanolen und Octanen.

Die Hydroformylierung kann auch in Gegenwart von Rhodiumkatalysatoren erfolgen. Solche Hydroformylierungsverfahren sind allgemein, so z. B. aus EP 0 213 639, EP 1 201 675, WO 03/16320, WO 03/16321, WO 2005/090276 und den dort zitierten Schriften bekannt. Spezielle Verfahren zur Hydroformylierung, die zur Herstellung von im erfindungsgemäßen Verfahren einsetzbaren Gemischen isomerer Nonanole ebenfalls geeignet sind, werden z. B. in WO 2004/020380 oder DE 103 27 435 beschrieben. Die dort beschriebenen Verfahren werden in Gegenwart von cyclischen Kohlensäureestern durchgeführt.

Es kann auch vorteilhaft sein, das Gemisch isomerer Octene vor der Zuführung zur Hydroformylierung zunächst wie in EP 1 172 349 beschrieben zu fraktionieren. Auf diese Weise ist es möglich, Octenfraktionen zu erhalten, die besonders gut zur Herstellung von im erfindungsgemäßen Verfahren einsetzbaren Gemischen isomerer Nonanole geeignet sind. Aus den Fraktionen kann dann auf relativ einfache Weise durch Mischen von geeigneten Fraktionen ein Gemisch von isomeren Octenen erhalten werden, welches zur Herstellung von Gemischen von isomeren Nonanolen zum Einsatz im erfindungsgemäßen Verfahren geeignet ist.

Die Hydroformylierung der Octengemische kann ein- oder mehrstufig, optional mit Abtrennung der nicht reagierten Octene nach jeder Stufe, durchgeführt werden. Optional kann und bevorzugt wird das aus der Hydroformylierung erhaltene Reaktionsgemisch fraktioniert und so die für die Hydrierung bestimmte Nonanalfraktion aufkonzentriert. In der Regel wird man allerdings das Hydroformylierungsprodukt direkt vom Katalysator befreien und danach der Hydrierung zuführen. Die Hydrierung erfolgt in der Regel an heterogenen Katalysatoren bei erhöhten Temperaturen und Drücken in Flüssig- oder Gasphasen-Fahrweise in an sich bekannter Weise, wie z. B. in WO 2009/027135 offenbart.

Geeignete Isononanolgemische im Sinne der vorliegenden Erfindung werden beispielsweise auch in EP1171413 genannt.

### Furandicarbonsäure

Furan-2,5-dicarbonsäure (FDCA, CAS-Nr: 3238-40-2) ein weißer Feststoff mit einem Schmelzpunkt > 300 °C, ist bisher nicht großtechnisch verfügbar, kann aber entweder gemäß Literaturangaben hergestellt oder kommerziell erworben werden. Die gegebenenfalls gewünschte oder bevorzugte Überführung in das Dichlorid wird in den Beispielen ausführlich beschrieben.

### Veresterung

Zur Herstellung der erfindungsgemäßen Ester wird entweder Furandicarbonsäure oder ein reaktives Derivat wie beispielsweise das entsprechende Dichlorid (s. Beispiele) mit einem Gemisch isomerer Nonanole umgesetzt. Bevorzugt erfolgt die Veresterung ausgehend von Furandicarbonsäure und Isononanol mit Hilfe eines Katalysators.

Die Veresterung der Furandicarbonsäure mit einem Isononanolgemisch zu den entsprechenden Estern kann autokatalytisch oder katalytisch, beispielsweise mit Brönstedt- oder Lewissäuren durchgeführt werden. Ganz gleich welche Art der Katalyse gewählt wird, es entsteht immer ein temperaturabhängiges Gleichgewicht zwischen den Einsatzstoffen (Säure und Alkohol) und den Produkten (Ester und Wasser). Um das Gleichgewicht zu Gunsten des Esters zu verschieben, kann ein Schleppmittel eingesetzt werden, mit dessen Hilfe das Reaktionswasser aus dem Ansatz entfernt wird. Da die zur Veresterung eingesetzten Alkoholgemische niedriger als die Furandicarbonsäure, deren reaktive Derivate und deren Ester sieden und mit Wasser eine Mischungslücke aufweisen, werden sie häufig als Schleppmittel eingesetzt, das nach Wasserabtrennung wieder in den Prozess zurückgeführt werden kann.

Der zur Bildung des Esters eingesetzte Alkohol bzw. das isomere Nonanolgemisch, das gleichzeitig als Schleppmittel dient, wird im Überschuss, bevorzugt 5 bis 50 Massen-%, insbesondere 10 bis 30 Massen-% der zur Bildung des Esters notwendigen Menge eingesetzt.

Als Veresterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirkonium, die als fein verteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität oft erst bei Temperaturen oberhalb 180 °C erreichen. Hierbei ist jedoch zu beachten, dass die Furandicarbonsäure bei Temperaturen oberhalb von 190 °C zur Abspaltung von CO₂ neigt, und sich hieraus dann die Monocarbonsäure bildet, welche nicht mehr zum Zielprodukt umgesetzt werden kann. Die Metallkatalysatoren werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat.

Die Katalysatorkonzentration hängt von der Art des Katalysators ab. Bei den bevorzugt eingesetzten Titanverbindungen beträgt diese 0,005 bis 2,0 Massen-% bezogen auf das Reaktionsgemisch, insbesondere 0,01 bis 0,5 Massen-%, ganz besonders 0,01 bis 0,1 Massen-%.

Die Reaktionstemperaturen liegen bei Verwendung von Titankatalysatoren zwischen 160 °C und 270 °C, vorzugsweise bei 160 bis 200 °C. Die optimalen Temperaturen hängen von den Einsatzstoffen, Reaktionsfortschritt und der Katalysatorkonzentration ab. Sie können für jeden Einzelfall durch Versuche leicht ermittelt werden. Höhere Temperaturen erhöhen die Reaktionsgeschwindigkeiten und begünstigen Nebenreaktionen, wie beispielsweise Wasserabspaltung aus Alkoholen oder Bildung farbiger Nebenprodukte. Es ist zur Entfernung des Reaktionswassers günstig, dass der Alkohol aus dem Reaktionsgemisch abdestillieren kann. Die gewünschte Temperatur oder der gewünschte Temperaturbereich kann durch den Druck im Reaktionsgefäß eingestellt werden. Bei niedrig siedenden Alkoholen wird daher die Umsetzung bei Überdruck und bei höher siedenden Alkoholen bei vermindertem Druck durchgeführt. Beispielsweise wird bei der Umsetzung von FDCA mit einem Gemisch isomerer Nonanole in einem Temperaturbereich von 160 °C bis 190 °C im Druckbereich von 0,1 MPa bis 0,001 MPa gearbeitet.

Die in die Reaktion zurückzuführende Flüssigkeitsmenge kann teilweise oder vollständig aus Alkohol bestehen, der durch Aufarbeitung des azeotropen Destillats gewonnen wird. Es ist auch möglich, die Aufarbeitung zu einem späteren Zeitpunkt durchzuführen und die entfernte Flüssigkeitsmenge ganz oder teilweise durch frischen Alkohol, d. h. aus einem im Vorratsgefäß bereit stehenden Alkohol zu ersetzen.

Die Rohestergemische, die neben dem/den Ester(n), Alkohol, Katalysator oder dessen Folgeprodukten und gegebenenfalls Nebenprodukte enthalten, werden nach an sich bekannten Verfahren aufgearbeitet. Die Aufarbeitung umfasst dabei folgende Schritte: Abtrennung des überschüssigen Alkohols und ggf. Leichtsieder, Neutralisation der vorhandenen Säuren, optional eine Wasserdampfdestillation, Umwandlung des Katalysators in einen leicht filtrierbaren Rückstand, Abtrennung der Feststoffe und gegebenenfalls eine Trocknung. Dabei können je nach angewendetem Aufarbeitungsverfahren die Reihenfolge dieser Schritte verschieden sein.

Optional kann das Gemisch der Diisononylester aus dem Reaktionsgemisch, gegebenenfalls nach Neutralisation des Ansatzes, destillativ abgetrennt werden.

### Umesterung

Alternativ können die erfindungsgemäßen Diisoonylester durch Umesterung eines Furan-2,5-dicarbonsäurediesters mit einem Isononanolgemisch gewonnen werden. Als Edukte werden Furan-2,5-dicarbonsäurediester eingesetzt, deren am O-Atom der Estergruppe gebundenen Alkylreste 1-8 C-Atome aufweisen. Diese Reste können aliphatisch, geradkettig oder verzweigt, alicyclisch oder aromatisch sein. Eine oder mehrere Methylengruppen dieser Alkyl-Reste können durch Sauerstoff substituiert sein. Es ist zweckmäßig, dass die dem Eduktester zugrunde liegenden Alkohole niedriger sieden als das eingesetzte Isononanolgemisch. Ein bevorzugter Einsatzstoff ist Furan-2,5-dicarbonsäuredimethylester.

Die Umesterung wird katalytisch, beispielsweise mit Brönstedt- oder Lewissäuren oder Basen, durchgeführt. Ganz gleich welcher Katalysator eingesetzt wird, es entsteht immer ein temperaturabhängiges Gleichgewicht zwischen den Einsatzstoffen (Dialkylester und Isononanolgemisch) und den Produkten (Diisononylestergemisch und freigesetzter Alkohol). Um das Gleichgewicht zu Gunsten des Diisononylestergemisches zu verschieben, wird der aus dem Eduktester entstehende Alkohol aus dem Reaktionsgemisch abdestilliert.

Es ist auch hier zweckmäßig, das Isononanolgemisch im Überschuss einzusetzen. Als Umesterungskatalysatoren können Säuren, wie beispielsweise Schwefelsäure, Methansulfonsäure oder p-Toluolsulfonsäure, oder Metalle oder deren Verbindungen eingesetzt werden. Geeignet sind z. B. Zinn, Titan, Zirkonium, die als fein verteilte Metalle oder zweckmäßig in Form ihrer Salze, Oxide oder löslichen organischen Verbindungen verwendet werden. Die Metallkatalysatoren sind im Gegensatz zu Protonensäuren Hochtemperaturkatalysatoren, die ihre volle Aktivität erst bei Temperaturen oberhalb 180 °C erreichen. Sie werden jedoch bevorzugt eingesetzt, weil sie im Vergleich zur Protonenkatalyse weniger Nebenprodukte, wie beispielsweise Olefine aus dem eingesetzten Alkohol, bilden. Beispielhafte Vertreter für Metallkatalysatoren sind Zinnpulver, Zinn(II)oxid, Zinn(II)oxalat, Titansäureester wie Tetraisopropylorthotitanat oder Tetrabutylorthotitanat sowie Zirkoniumester wie Tetrabutylzirkonat.

Weiterhin können basische Katalysatoren, wie beispielsweise Oxide, Hydroxide, Hydrogencarbonate, Carbonate oder Alkoholate von Alkali- oder Erdalkalimetallen verwendet werden. Aus dieser Gruppe werden bevorzugt Alkoholate, wie beispielsweise Natriummethylat eingesetzt. Alkoholate können auch in situ aus einem Alkalimetall und einem Nonanol bzw. einem Isononanolgemisch hergestellt werden.

Die Katalysatorkonzentration hängt von der Art des Katalysators ab. Sie liegt üblicherweise zwischen 0,005 bis 2,0 Massen-% bezogen auf das Reaktionsgemisch.

Die Reaktionstemperaturen für die Umesterung liegen üblicherweise zwischen 100 und 220 °C. Sie müssen mindestens so hoch sein, dass der aus dem Eduktester entstehende Alkohol bei dem vorgegebenen Druck, meistens Normaldruck, aus dem Reaktionsgemisch abdestillieren kann.

Die Umesterungsgemische können genauso wie für die Veresterungsgemische beschrieben aufgearbeitet werden.

### Verwendung

Die erfindungsgemäßen Gemische isomerer Nonylester der 2,5-Furandicarbonsäure können als Weichmacher, insbesondere in Kunststoffzusammensetzungen, Klebstoffen, Dichtungsmassen, Lacken, Farben, Plastisolen, Kunstledern, Fußbodenbelägen, Unterbodenschutz, beschichteten Geweben, Tapeten oder Tinten verwendet werden. Vorzugsweise können die erfindungsgemäßen Weichmacher in Profilen, Dichtungen, Lebensmittelverpackungen, Folien, Spielzeugen, Medizinalartikeln, Dachbahnen, Kunstledern, Fußbodenbelägen, Unterbodenschutz, beschichteten Geweben, Tapeten, Kabeln und Drahtummantelungen, besonders bevorzugt in Lebensmittelverpackungen, Spielzeugen, Medizinalartikeln, wie z. B. in Beuteln und Schlauchmaterial für Infusionen, Dialyse und Drainagen, Tapeten, Fußbodenbelägen und beschichteten Geweben verwendet werden.

Unter Verwendung der erfindungsgemäßen Gemische isomerer Nonylester der 2,5-Furandicarbonsäure sind insbesondere erfindungsgemäße Zusammensetzungen erhältlich, die das Gemisch isomerer Nonylester der 2,5-Furandicarbonsäure enthalten.

Solche Zusammensetzungen können das erfindungsgemäße Gemisch isomerer Nonylester der 2,5-Furandicarbonsäure alleine oder in Mischungen mit anderen Weichmachern aufweisen. Falls die erfindungsgemäßen Zusammensetzungen das erfindungsgemäße Gemisch isomerer Nonylester der 2,5-Furandicarbonsäure im Gemisch mit anderen Weichmachern aufweisen, so können die anderen Weichmacher vorzugsweise aus der Gruppe der Phthalsäuredialkylester, bevorzugt mit 4 bis 13 C-Atomen in der Alkylkette; Trimellitsäuretrialkylester, bevorzugt mit 4 bis 10 C-Atomen in der Seitenkette; Adipinsäuredialkylester und bevorzugt Terephthalsäuredialkylester jeweils bevorzugt mit 4 bis 13 C-Atomen in der Seitenkette; 1,2-Cyclohexandisäurealkylestern, 1,3-Cyclohexandisäurealkylestern und 1,4-Cyclohexandisäurealkylestern, bevorzugt 1,2-Cyclohexandisäurealkylestern, jeweils bevorzugt mit Alkyl = Alkylrest mit 4 bis 13 Kohlenstoffatomen in der Seitenkette; Dibenzoesäurester von Glykolen; Alkylsulfonsäurester von Phenol mit vorzugsweise einem Alkylrest, der 8 bis 22 C-Atome enthält; Polymerweichmacher, Glycerinester, Isosorbidester und Benzoesäurealkylester, vorzugsweise mit 7 bis 13 C-Atomen in der Alkylkette, ausgewählt sein. In allen Fällen können die Alkylreste linear oder verzweigt sowie gleich oder verschieden sein. Besonders bevorzugt weist die Zusammensetzung neben dem Gemisch isomerer Nonylester der 2,5-Furandicarbonsäure insbesondere einen Benzoesäurealkylester mit Alkyl = Alkylrest mit 7 bis 13 Kohlenstoffatomen, vorzugsweise Benzoesäureisononylester, Benzoesäurenonylester, Benzoesäureisodecylester, Benzoesäurepropylheptylester oder Benzoesäuredecylester auf. Der Anteil an erfindungsgemäßen Gemischen isomerer Nonylester der 2,5-Furandicarbonsäure in dem Gemisch mit anderen Weichmachern beträgt vorzugsweise 15 bis 90 Massen-%, besonders bevorzugt 20 bis 80 Massen-% und ganz besonders bevorzugt 30 bis 70 Massen-%, wobei sich die Massenanteile aller vorhandenen Weichmacher zu 100 Massen-% addieren.

Die genannten Zusammensetzungen aus Gemischen isomerer Nonylester der 2,5-Furandicarbonsäure und anderen Weichmachern können als Weichmacherzusammensetzung in Kunststoffzusammensetzungen, Klebstoffen, Dichtungsmassen, Lacken, Farben, Plastisolen oder Tinten verwendet werden. Aus den erfindungsgemäßen Weichmacherzusammensetzungen hergestellte Kunststoffprodukte können beispielsweise sein: Profile, Dichtungen, Lebensmittelverpackungen, Folien, Spielzeug, Medizinalartikel, Dachbahnen, Kunstleder, Fußbodenbeläge, Unterbodenschutz, beschichtete Gewebe, Tapeten, Kabel und Drahtummantelungen. Bevorzugt sind aus dieser Gruppe Lebensmittelverpackungen, Spielzeug, Medizinalartikel, Tapeten, beschichtete Gewebe und Fußbodenbeläge zu nennen.

Die erfindungsgemäßen Zusammensetzungen, die ein Gemisch isomerer Nonylester der 2,5-Furandicarbonsäure enthalten, können ein Polymer, ausgewählt aus Polyvinylchlorid (PVC), Polyvinylidenchlorid (PVDC), Polyacrylaten, insbesondere Polymethylmethacrylat (PMMA), Polyalkylmethacrylat (PAMA), Fluorpolymeren, insbesondere Polyvinylidenfluorid (PVDF), Polytetrafluorethylen (PTFE), Polyvinylacetat (PVAc, Polyvinylalkohol (PVA), Polyvinylacetale, insbesondere Polyvinylbutyral (PVB), Polystyrolpolymere, insbesondere Polystyrol (PS), Expandierbares Polystyrol (EPS), Acrylonitril-Styrol-Acrylat (ASA), Styrolacrylonitril (SAN), Acrylonitril-Butadien-Styrol (ABS), Styrol-Maleinsäureanhydrid-Copolymer (SMA), Styrol-Methacrylsäure-Copolymer, Polyolefine, insbesondere Polyethylen (PE) oder Polypropylen (PP), thermoplastische Polyolefine (TPO), Polyethylen-Vinylacetat (EVA), Polycarbonate, Polyethylenterephthalat (PET), Polybutylenterephthalat (PBT), Polyoxymethylen (POM), Polyamid (PA), Polyethylenglykol (PEG), Polyurethan (PU), Thermoplastisches Polyurethan (TPU), Polysulfide (PSu), Biopolymere, insbesondere Polymilchsäure (PLA), Polyhydroxybutyral (PHB), Polyhydroxyvaleriansäure (PHV), Polyester, Stärke, Cellulose und CelluloseDerivate, insbesondere Nitrocellulose (NC), Ethylcellulose (EC), Celluloseacetat (CA), Cellulose-Acetat/Butyrat (CAB), Gummi oder Silikone sowie Mischungen oder Copolymere der genannten Polymere oder deren monomeren Einheiten aufweisen. Vorzugsweise weisen die erfindungsgemäßen Zusammensetzungen PVC oder Homo-oder Copolymere auf Basis von Ethylen, Propylen, Butadien, Vinylacetat, Glycidylacrylat, Glycidylmethacrylat, Methacrylaten, Acrylaten, Acrylaten oder Methacrylaten mit am Sauerstoffatom der Estergruppe gebundenen Alkylresten von verzweigten oder unverzweigten Alkoholen mit einem bis zehn Kohlenstoffatome(n), Styrol, Acrylnitril oder cyclischen Olefinen auf.

Bevorzugt enthält die erfindungsgemäße Zusammensetzung als PVC-Typ Suspensions-, Masse-, Mikrosuspensions- oder Emulsions-PVC. Bezogen auf 100 Massenteile Polymer enthalten die erfindungsgemäßen Zusammensetzungen vorzugsweise von 5 bis 200, bevorzugt von 10 bis 150 Massenteile an Weichmacher.

Die erfindungsgemäßen Zusammensetzungen können neben den genannten Bestandteilen weitere Bestandteile enthalten, insbesondere z. B. weitere Weichmacher, Füllstoffe, Pigmente, Stabilisatoren, Co-Stabilisatoren wie beispielsweise epoxidiertes Sojabohnenöl sowie Gleitmittel, Treibmittel, Kicker, Antioxidanzien oder Biozide.

Die erfindungsgemäßen Zusammensetzungen liegen bevorzugt als Flüssigkeit, insbesondere als pumpbare Flüssigkeit, als Paste, Schutzmasse, Plastisol, Pulver, Feststoff oder Festkörper vor.

Die genannten, Zusammensetzungen, welche besagte Polymere aufweisen, können als Klebstoffe, Dichtungsmassen, Lacke, Farben, Plastisole, Kunstleder, Fußbodenbeläge, Unterbodenschutz, Gewebebeschichtungen, Tapeten oder Tinten oder zu deren Herstellung verwendet werden.

Soweit es sich bei den genannten Zusammensetzungen um Kunststoffe handelt, können diese zu Profilen, Dichtungen, ein- oder mehrteilige Verschlussvorrichtungen, Lebensmittelverpackungen, Folien, Spielzeug, Medizinalartikel, insbesondere Beuteln und Schlauchmaterial, wie sie z. B. für Infusionen, Dialyse und Drainagen verwendet werden, Dachbahnen, Kunstleder, Fußbodenbeläge, Unterbodenschutz, beschichtete Gewebe, Tapeten, Kabel und Drahtummantelungen verarbeitet werden. Bevorzugt werden die erfindungsgemäßen Zusammensetzungen für die Herstellung von Lebensmittelverpackungen, Spielzeug, Medizinalartikel, Tapeten und Fußbodenbelägen verwendet.

Die folgenden Beispiele sollen die Erfindung erläutern, ohne ihre Anwendungsbreite einzuschränken, die sich aus der Beschreibung und den Ansprüchen ergibt. Auch ohne weitere Ausführungen wird davon ausgegangen, dass der Fachmann die vorliegende Erfindung im weitesten denkbaren Rahmen nutzen kann.

### Beispiele

Die erfindungsgemäßen Ester wurden in einer zweistufigen Synthese ausgehend von Furan-2,5-dicarbonsäure über das Dichlorid hergestellt.

### Beispiel 1: Synthesevorschrift für Furan-2,5-dicarbonsäuredichlorid (II)

In einem 250 mL-Dreihalskolben, mit Rückflusskühler und Tropftrichter wurden unter Argon 72,1 g (462 mmol) Furan-2,5-dicarbonsäure vorgelegt. In einem Zeitraum von 10 min wurden 165 g (1.39 mol) Thionylchlorid, versetzt mit einigen Tropfen N,N-Dimethylformamid, zugegeben. Die Suspension wurde auf Rückfluss-Temperatur erhitzt und das entstehende Gas durch Waschflaschen mit wässriger KOH-Lösung abgeleitet. Es wurde dann 4 h bis zur Beendigung der Gasentwicklung und vollständigen Auflösung des Feststoffes unter Rückfluss erhitzt.

Die Isolierung des Produktes erfolgte, nach Abziehen von überschüssigem Thionylchlorid, durch destillative Aufreinigung (T = 110 °C, p = 0,0012 MPa).

Hierbei resultierten 79,4 g Dichlorid als farbloser, kristalliner Feststoff (Ausbeute 89 %) mit einem Schmelzpunkt: von 79,5 - 80,0 °C

Furan-2,5-dicarbonsäuredichlorid wurde bis zur Weiterverwendung unter Schutzgas (Argon) im Dunklen bei Raumtemperatur gelagert.

### Beispiel 2: Synthese der Furan-2,5-dicarbonsäureester

Unter Argon wurde in einem Dreihalskolben mit Rückflusskühler und Tropftrichter das Dichlorid vorgelegt und durch Erhitzen geschmolzen. Zur Flüssigkeit wurden 2,4 Äquivalente Alkohol langsam zugetropft, wobei es zur exothermen Reaktion mit Gasentwicklung kam. Das entstehende Gas wurde durch Waschflaschen mit wässriger KOH-Lösung geleitet. Nach vollständiger Zugabe wurde 16 h bei einer Temperatur von 80 - 100 °C gerührt.

Der überschüssige Alkohol wurde in Gegenwart von Siedesteinen unter reduziertem Druck entfernt und das Rohprodukt destillativ aufgereinigt.

Für die Synthese des Vergleichsbeispiels wurde kommerziell erhältliches 2-Ethylhexanol eingesetzt. Zur Herstellung des erfindungsgemäßen Estergemisches wurde kommerziell unter dem Produktnamen Isononanol INA erhältliches Isononanol des Anmelders, CAS Reg. Nr. 27458-94-2, verwendet. Das verwendete Isononanol weist eine Dichte (gemäß DIN 51757) bei 20°C von 0,8348 g/cm³, einen Brechungsindex (gemäß DIN 51423/2) bei 20 °C von 1,4362 und eine Scherviskosität (gemäß DIN 53015) bei 20 °C von 13,2 mPa*s, sowie einen Erstarrungspunkt < -75 °C auf, und hatte die folgende Zusammensetzung; ermittelt gemäß gaschromatographischer Analyse: 7,5 Mol% n-Nonanol; 19,8 Mol% 6-Methyloctanol; 20,0 Mol% 4-Methyloctanol; 3,8 Mol-% 2-Methyloctanol; 8,3 Mol% 3-Ethylheptanol; 2,1 Mol% 2-Ethylheptanol; 1,8 Mol% 2-Propylhexanol; 15,0 Mol% 4,5-Dimethylheptanol; 10,1 Mol% 2,5-Dimethylheptanol; 2,5 Mol-% 2,3-Dimethylheptanol; 4,1 Mol% 3-Ethyl-4-methylhexanol; 2,9 Mol% 2-Ethyl-4-methylhexanol; 2,1 Mol% sonstige nicht identifizierte Verbindungen mit 9 Kohlenstoffatomen; die Gesamtsumme der genannten Komponenten ergab 100 Mol%. In der nachfolgenden Tabelle 1 sind die Ergebnisse der beiden Synthesen dokumentiert.

**Tabelle 1:**

| **Ester** | **Siedepunkt Ester** | **Ausbeute** |
|---|---|---|
| Furan-2,5-dicarbonsäure-bis-(2-ethyl-hexyl)-ester **II (Vergleichsbeispiel)** | 137 - 138 °C (p = 0,0002·MPa) | 99 % |
| Furan-2,5-dicarbonsäure-bis-(isononyl)-ester (**I**) **(erfindungsgemäß)** | 155 - 185 °C (p = 0,0004-MPa) | 98 % |

Die Umsetzungen von Furan-2,5-dicarbonsäuredichlorid (2) zu den korrespondierenden Estern erfolgen somit nahezu quantitativ.

### Beispiel 3: Bestimmung des Verhaltens der Ester in der Kälte mittels Differential Scanning Calorimetry (DSC)

### Gerät: DSC820 der Firma Mettler Toledo

### Untersuchungsbedingungen:

| | |
|---|---|
| Temperaturbereich: | -100 bis 250 °C |
| Heizrate: | 10 K/min |
| Einwaage: | ca. 10 - 11 mg |
| Tiegel: | Standard-Aluminium-Tiegel mit Löchern im Deckel |
| Spülgas: | N2 |

Ergebnis: Während der 2-EH-Ester **II** (Vergleichsbeispiel) ein Schmelzsignal oberhalb von 0 °C zeigt, d.h. bereits oberhalb des Gefrierpunktes als Feststoff vorliegt, erstarrt der erfindungsgemäße Isononylester **I** glasartig bei ca. - 80 °C. Im DSC-Thermogramm sind keine Schmelzsignale sondern nur ein Glasübergangspunkt bei ca. -80 °C zu erkennen.

Somit kann davon ausgegangen werden, dass der erfindungsgemäße Ester I auch bei tiefen Temperaturen nicht fest wird, sondern fließ- bzw. pumpfähig bleibt.

### Beispiel 4: Herstellung von Plastisolen

Die mit den erfindungsgemäßen Estern erzielbaren vorteilhaften Eigenschaften sollen im Folgenden an Plastisolen und hieraus erhältlichen Halbzeugen aufgezeigt werden.

Die verwendeten Einwaagen der Komponenten für die verschiedenen Plastisole sind der nachfolgenden Tabelle 2 zu entnehmen.

**Tabelle 2: Rezepturen [Alle Angaben in phr (= Massenteile pro 100 Massenteile PVC)]**

| **Plastisolrezeptur** | **1** | **2** | **3** |
|---|---|---|---|
| Emulsions-PVC (Vestolit B 7021 Ultra der Fa. Vestolit GmbH) | 100 | 100 | 100 |
| Furan-2,5-dicarbonsäurediisononylester **I** (erfindungsgemäß) | 50 | | |
| Furan-2,5-dicarbonsäuredi-2-ethylhexylester **II** (Vergleichsbeispiel) | | 50 | |
| DINP (VESTINOL 9 der Evonik Oxeno GmbH, Vergleichsbeispiel) | | | 50 |
| Epoxidiertes Sojabohnenöl (Drapex 39, Fa. Chemtura) | 3 | 3 | 3 |
| Ca/Zn-Stabilisator (Mark CZ 149, Fa. Chemtura) | 2 | 2 | 2 |

Die flüssigen Bestandteile wurden vor den festen Bestandteilen in einem geeigneten PE-Becher eingewogen. Von Hand wurde die Mischung mit einem Salbenspatel so eingerührt, dass kein unbenetztes Pulver mehr vorhanden war. Der Mischbecher wurde dann in die Klemmvorrichtung eines Dissolverrührers eingespannt. Mit einer Mischerscheibe wurde die Probe homogenisiert.

Dabei wurde die Drehzahl von 330 U/min bis 2000 U/min erhöht, und so lange gerührt, bis die Temperatur an der Digitalanzeige des Thermofühlers 30,0 °C erreichte. Damit war sicher gestellt, dass die Homogenisierung des Plastisols bei einem definierten Energieeintrag erreicht wurde. Danach wurde das Plastisol sofort bei 25,0 °C temperiert.

### Beispiel 5: Messung der Plastisolviskosität

Die Messung der Viskositäten der in Beispiel 4 hergestellten Plastisole wurden mit einem Rheometer Physica MCR 101 (Fa. Paar-Physica), welches über die zugehörige Software Rheoplus gesteuert wurde, wie folgt durchgeführt.

Das Plastisol wurde im Vorratsbehälter nochmals mit einem Spatel umgerührt und in dem Messsystem Z3 (DIN 25 mm) gemäß Bedienungsanleitung vermessen. Die Messung verlief bei 25 °C automatisch über die o. g. Software. Folgende Punkte wurden angesteuert.

Eine Vorscherung von 100 s⁻¹ für den Zeitraum von 60 s, bei der keine Messwerte aufgenommen wurden (Nivellierung thixotroper Effekte).

Eine isotherme Abwärtsrampe, beginnend bei einer Schergeschwindigkeit von 200 s⁻¹ bis herunter zu 0,1 s⁻¹, aufgeteilt in eine logarithmische Reihe mit 30 Schritten mit jeweils 5 s Messpunktdauer.

Die Aufbereitung der Messdaten wurde nach der Messung automatisch von der Software durchgeführt. Dargestellt wurde die Viskosität in Abhängigkeit von der Schergeschwindigkeit. Um Veränderungen der Plastisolviskosität während der Plastisollagerung (auch: "Plastisolreifung") zu erfassen, wurden die Messungen jeweils nach 2 h, 24 h und 7 Tagen durchgeführt. Zwischen diesen Zeitpunkten wurden die Plastisole bei 25 °C gelagert.

In der nachfolgenden Tabelle sind exemplarisch für die Schergeschwindigkeit von 100 s⁻¹ jeweils die nach den angegebenen Lagerzeiten erhaltenen entsprechenden Viskositätswerte aufgeführt.

**Tabelle 3: Plastisolviskositäten bei einer Schergeschwindigkeit von 100 s⁻¹**

| Plastisol-Nr. | Verwendeter Weichmacher | Viskosität nach 2 h in Pa * s | Viskosität nach 24 h in Pa * s | Viskosität nach 7 Tagen in Pa * s | Prozentualer Anstieg in % |
|---|---|---|---|---|---|
| 1 | Furan-2,5-dicarbonsäurediisononylester **I** (erfindungsgemäß) | 10,3 | 10,6 | 11,6 | 13 |
| 2 | Furan-2,5-dicarbonsäuredi-2-ethylhexylester **II** (Vergleichsbeispiel) | 9,0 | 9,4 | 11,1 | 23 |
| 3 | DINP (Vergleichsbeispiel) | 5,9 | 6,14 | 6,5 | 10 |

Die erfindungsgemäßen Isononylester I zeigen gegenüber den 2-Ethylhexylestern **II** deutlich geringere Anstiege in der Viskosität der Plastisole mit der Zeit. Das im Vergleich mit dem Standardweichmacher DINP in der vorliegenden Formulierung höhere Viskositätsniveau des erfindungsgemäßen PVC-Plastisols kann, wie dem Fachmann bekannt ist, in (optimierten) Formulierungen und/oder anderen Zusammensetzungen durch geeignete Maßnahmen wie z. B. die Erhöhung der Gesamtweichmachermenge, die Zugabe von zusätzlichen Weichmachern mit niedrigerer Eigenviskosität, der Zugabe von Rheologieadditiven (z. B. Dispergieradditiven oder anderen oberflächenaktiven Substanzen) und/oder der Zugabe von (Co)-Lösungsmitteln abgesenkt werden.

### Beispiel 6: Messung der Geliergeschwindigkeit

Die Untersuchung des Gelierverhaltens der Plastisole wurde im Physica MCR 101 im Oszillationsmodus mit einem Platte-Platte Meßsystem (PP25), welches schubspannungsgesteuert betrieben wurde, vorgenommen. Eine zusätzliche Temperrierhaube wurde an das Gerät angeschlossen, um eine homogene Wärmeverteilung und eine gleichmäßige Probentemperatur zu erreichen.

Folgende Parameter wurden eingestellt:
Modus:
   Temperatur-Gradient
   Start-Temperatur: 25 °C
   End-Temperatur: 180 °C
   Heiz/Kühlrate: 5 °C/min
   Oszillations-Frequenz: 4-0,1 Hz Rampe logarithmisch
   Kreisfrequenz Omega: 10 1/s
   Anzahl Messpunkte: 63
   Messpunktdauer: 0,5 min
   Automatische Spaltnachführung F : 0 N
   Konstante Messpunktdauer
   Spaltweite 0,5 mm

### Durchführung der Messung:

Auf die untere Messsystemplatte wurde mit dem Spatel ein Tropfen der zu messenden Plastisolrezeptur luftblasenfrei aufgetragen. Dabei wurde darauf geachtet, dass nach dem Zusammenfahren des Messsystems etwas Plastisol gleichmäßig aus dem Messsystem herausquellen konnte (nicht mehr als ca. 6 mm rundum). Anschließend wurde die Temperierhaube über der Probe positioniert und die Messung gestartet.

Bestimmt wurde die sog. komplexe Viskosität des Plastisols in Abhängigkeit von der Temperatur. Da eine bestimmte Temperatur in einer (durch die Heizrate von 5 °C/min. festgelegte) Zeitspanne erreicht wird, wird neben der Geliertemperatur auch eine Aussage zur Geliergeschwindigkeit des vermessenen Systems erhalten. Ein Einsetzen des Geliervorganges war in einem plötzlichen starken Anstieg der komplexen Viskosität zu erkennen. Je früher dieser Viskositätsanstieg einsetzt, desto besser ist die Gelierfähigkeit des Systems.

Für einen Vergleich wurde aus den Kurven durch Interpolation für jedes Plastisol die Temperatur bestimmt, bei der eine komplexe Viskosität von 1000 Pa · s erreicht war.

Hierbei ergaben sich die in Tabelle 4 aufgeführten Werte:

**Tabelle 4: Gelierverhalten**

| Plastisole gemäß Bespiel 4 | 1 (erfindungsgemäß) | 2 (Vergleichsbeispiel) | 3 (Vergleichsbeispiel) |
|---|---|---|---|
| Temperatur bei Viskosität 1000 Pa _{*} s | 85 °C | 80,5 °C | 88,5 °C |

Es ist deutlich zu erkennen, dass die Furandicarbonsäureester früher (d. h. bei niedrigeren Temperaturen) gelieren als die entsprechenden Phthalate.

### Beispiel 7: Messung der Shore-Härte von Gießlingen

Die Shore-Härte A ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Umgekehrt kann bei sehr effizienten Weichmachern ein gewisser Anteil in der Rezeptur eingespart werden, was in vielen Fällen für den Verarbeiter geringere Kosten bedeutet.

Zur Bestimmung der Shore-Härten wurden die gemäß Beispiel 4 hergestellten Plastisole in kreisrunde Gießformen mit einem Durchmesser von 42 mm gegossen. Dann wurden die Plastisole in den Formen im Umlufttrockenschrank 30 min bei 200 °C geliert, nach Abkühlung entnommen und vor der Messung mindestens 24 Stunden bei 25 °C gelagert. Die Dicke der Gießlinge betrug ca. 12 mm.

Die Messungen selbst wurden nach DIN 53 505 mit einem Shore-A-Messgerät der Fa. Zwick-Roell durchgeführt, der Messwert jeweils nach 3 Sekunden abgelesen. An jedem Probekörper wurden drei verschiedene Messungen an verschiedenen Stellen (nicht im Randbereich) durchgeführt und jeweils der Mittelwert notiert.

In Tabelle 5 sind die erhaltenen Messwerte aufgeführt.

**Tabelle 5: Shore-A-Härten**

| Plastisole gemäß Bespiel 4 | 1 (erfindungsgemäß) | 2 (Vergleichsbeispiel) | 3 (Vergleichsbeispiel) |
|---|---|---|---|
| Shore-Härte A | 78 | 75 | 80 |

Die aufgeführten Beispiele belegen, dass der erfindungsgemäße Di-isononylester der Furandicarbonsäure **I** gegenüber dem nächstliegenden Stand der Technik, dem Furan-2,5-dicarbonsäure-bis-(2-ethyl-hexyl)-ester **II**, den entscheidenden Vorteil der Nicht-Kristallisation aufweist. Gegenüber dem entsprechenden Phthalat DINP zeigen sich teilweise deutliche Verbesserungen bei der weichmachenden Wirkung und der Geliergeschwindigkeit.

### Beispiel 8:

### Verwendung der erfindungsgemäßen Furandicarbonsäureester in PVC-Deckstrichformulierung (Plastisol) zusammen mit Diisononylterephthalat (DINT) - Herstellung der Deckstrich-Plastisole.

Die Herstellung der Plastisole erfolgte gemäß Beispiel 4 jedoch mit veränderter Rezeptur. Die verwendeten Einwaagen der Komponenten für die verschiedenen Plastisole sind der nachfolgenden Tabelle (6) zu entnehmen.

**Tabelle 6: Rezepturen**

| [Alle Angaben in phr (= Massenteile pro 100 Massenteile PVC)] | | | |
|---|---|---|---|
| **Plastisolrezeptur** | **1** | **2** | **3** |
| Mikro Suspensions-PVC K70 (VESTOLIT B7021 Ultra, Fa. Vestolit) | 100 | 100 | 100 |
| Furan-2,5-dicarbonsäurediisononylester; Herstellung gemäß Beispiel 2 (erfindungsgemäß) | 10 | 15 | |
| Diisononyl(ortho)phthalat [DINP] (VESTINOL 9 der Evonik Oxeno GmbH, Vergleichsbeispiel) | | | 50 |
| Diisononylterephthalat (Laborprodukt, Herstellung gemäß DE 102008006400A1 / Beispiel 1) | 40 | 35 | |
| Epoxidiertes Sojabohnenöl (Drapex 39; Fa. Chemtura / Galata) | 3 | 3 | 3 |
| Calcium/Zink-Stabilisator (Mark CZ 149, Fa. Chemtura / Galata) | 2 | 2 | 2 |

### Beispiel 9:

### Bestimmung der Plastisolviskosität der Deckstrichplastisole (gemäß Beispiel 8) enthaltend die erfindungsgemäßen Furandicarbonsäureestern und Diisononylterephthalat nach einer Lagerdauer von 24 h (bei 25°C).

Die Messung der Viskositäten der in Beispiel 8 hergestellten Plastisole erfolgte mit einem Rheometer Physica MCR 101 (Fa. Paar-Physica), gemäß der in Beispiel 5 beschriebenen Vorgehensweise. Die Ergebnisse werden in der nachfolgenden Tabelle (7) exemplarisch für die Schergeschwindigkeiten 100/s, 10/s, 1/s und 0,1/s dargestellt.

**Tabelle 7: Scherviskosität der Plastisole aus Beispiel 8 nach 24h Lagerung bei 25 °C.**

| **Plastisolrezeptur (gemäß Bsp. 8)** | **1*** | **2*** | **3**** |
|---|---|---|---|
| Scherviskosität bei Schergeschwindigkeit= 100/s [Pa*s] | 8 | 8 | 6 |
| Scherviskosität bei Schergeschwindigkeit= 10/s [Pa*s] | 3,6 | 3,5 | 3,1 |
| Scherviskosität bei Schergeschwindigkeit= 1/s [Pa*s] | 2,7 | 2,9 | 2,8 |
| Scherviskosität bei Schergeschwindigkeit= 0,1/s [Pa*s] | 3,1 | 3,4 | 3,7 |

| | | | |
|---|---|---|---|
| ** = Vergleichsbeispiel * = Erfindungsgemäß | | | |

Im Bereich niedriger Schergeschwindigkeiten liegen die die erfindungsgemäßen Furandicarbonsäureester enthaltenden Plastisole in ihrer Scherviskosität unterhalb, bzw. auf dem Niveau des analogen DINP-Plastisols. Bei höheren Schergeschwindigkeiten liegen die Scherviskositäten der erfindungsgemäßen Plastisole lediglich geringfügig über der Scherviskosität des analogen DINP-Plastisols. Durch Abmischung von Diisononylterephthalat mit den erfindungsgemäßen Furandicarbonsäureestern lassen sich somit Plastisole herstellen, die ähnliche Verarbeitungseigenschaften wie DINP-Plastisole aufweisen, jedoch gleichzeitig keine Ortho-Phthalate enthalten, und auf nachwachsenden Rohstoffen basieren.

### Beispiel 10:

### Verwendung der erfindungsgemäßen Furandicarbonsäureester in thermisch expandierbaren Plastisolen (Fußboden) zusammen mit Schnellgelierern - Herstellung der Plastisole.

Die Herstellung der Plastisole erfolgte gemäß Beispiel 4 jedoch mit veränderter Rezeptur. Die verwendeten Einwaagen der Komponenten für die verschiedenen Plastisole sind der nachfolgenden Tabelle (8) zu entnehmen.

**Tabelle 8: Rezepturen thermisch expandierbarer Plastisole.**

| [Alle Angaben in phr (= Massenteile pro 100 Massenteile PVC)] | | | | | | |
|---|---|---|---|---|---|---|
| **Plastisolrezeptur** | **1**** | **2*** | **3*** | **4*** | **5*** | **6*** |
| Vinnolit MP 6852 | 100 | 100 | 100 | 100 | 100 | 100 |
| Vestinol 9 | 50 | 12,5 | | | | |
| Citrofol BII | | | 12,5 | | | |
| Mesamol II | | | | 12,5 | | |
| Jayflex MB10 | | | | | 12,5 | |
| Eastman DBT | | | | | | 12,5 |
| DINFDC | | 37,5 | 37,5 | 37,5 | 37,5 | 37,5 |
| Unifoam AZ Ultra 7043 | 3 | 3 | 3 | 3 | 3 | 3 |
| ZnO | 0,7 | 0,7 | 0,7 | 0,7 | 0,7 | 0,7 |

| | | | | | | |
|---|---|---|---|---|---|---|
| ** = Vergleichsbeispiel * = Erfindungsgemäß | | | | | | |

Die verwendeten Stoffe und Substanzen werden im Folgenden näher erläutert:
**Vinnolit MP 6852:** Mikrosuspensions PVC (Homopolymer) mit K-Wert (gemäß DIN EN ISO 1628-2) von 68; Fa. Vinnolit GmbH & Co KG.
**Vestinol 9:** Diisononyl(ortho)phthalat [DINP], Weichmacher; Fa. Evonik Oxeno GmbH.
**Citrofol BII:** Acetyltributylcitrat, Weichmacher mit schneller Gelierung; Fa. Jungbunzlauer AG.
**Mesamol** II: Alkylsulfonsäureester des Phenols; Weichmacher mit schneller Gelierung; Fa. Lanxess AG.
**Jayflex MB10:** Isodecylbenzoat; Weichmacher mit schneller Gelierung; Fa. ExxonMobil Chemicals.
**Eastman DBT:** Dibutylterephthalat; Weichmacher mit schneller Gelierung; Fa. Eastman Chemical Co.
**DINFDC:** erfindungsgemäßer Furan-2,5-dicarbonsäurediisononylester; Herstellung gemäß Beispiel 2.
**Unifoam AZ Ultra 7043:** Azodicarbonamid; Thermisch aktivierbares Treibmittel; Fa. Hebron S.A.
**ZnO:** Zinkoxid; Zersetzungskatalysator für thermisches Treibmittel; setzt die substanzeigene Zersetzungstemperatur des Treibmittels herab; Zinkoxid aktiv; Fa. Lanxess AG.

### Beispiel 11:

### Bestimmung der Plastisolviskosität der thermisch expandierbaren Plastisole (gemäß Beispiel 10) enthaltend die erfindungsgemäßen Furandicarbonsäureestern und Diisononyl(ortho)phthalat bzw. Schnellgelierern nach einer Lagerdauer von 24 h (bei 25 °C).

Die Messung der Viskositäten der in Beispiel 10 hergestellten Plastisole erfolgte mit einem Rheometer Physica MCR 101 (Fa. Paar-Physica), gemäß der in Beispiel 5 beschriebenen Vorgehensweise. Die Ergebnisse werden in der nachfolgenden Tabelle (9) exemplarisch für die Schergeschwindigkeiten 100/s, 10/s, 1/s und 0,1/s dargestellt.

**Tabelle 9: Scherviskosität der Plastisole aus Beispiel 8 nach 24h Lagerung bei 25 °C.**

| **Plastisolrezeptur (gemäß Bsp. 10)** | **1**** | **2*** | **3*** | **4*** | **5*** | **6*** |
|---|---|---|---|---|---|---|
| Scherviskosität bei Schergeschwindigkeit= 100/s [Pa*s] | 6,1 | 10,9 | 11,5 | 11,2 | 6,1 | 11 |
| Scherviskosität bei Schergeschwindigkeit= 10/s [Pa*s] | 4,5 | 7,8 | 8,5 | 10,2 | 4,7 | 9,2 |
| Scherviskosität bei Schergeschwindigkeit= 1/s [Pa*s] | 5,2 | 8,5 | 9,6 | 10,7 | 5,7 | 11,7 |
| Scherviskosität bei Schergeschwindigkeit= 0,1/s [Pa*s] | 8,2 | 12,9 | 15,2 | 15,7 | 9,5 | 22,5 |

| | | | | | | |
|---|---|---|---|---|---|---|
| ** = Vergleichsbeispiel * = Erfindungsgemäß | | | | | | |

Durch die Wahl des Schnellgelierers ist die Viskosität des Plastisols gezielt einstellbar, wobei im vorliegenden Fall die Kombination aus erfindungsgemäßem Furandicarbonsäurediisononlyester und Alkylbenzoat (Plastisolrezeptur 5) zu einem ähnlichen rheologischen Verhalten wie bei der Verwendung des Universalweichmachers Diisononyl(ortho)phthalat führt. D. h. es werden erfindungsgemäße Plastisole bereitgestellt, die unter ähnlichen Verarbeitungsbedingungen (z. B. Auftragsgeschwindigkeiten) wie der derzeitige Standardweichmacher DINP eingesetzt werden können, dabei aber keine Orthophthalate enthalten (müssen) und zumindest teilweise auf nachwachsenden Rohstoffen beruhen.

### Beispiel 12:

### Herstellung von Schaumfolien aus und Bestimmung des Expansions- bzw. Aufschäumverhaltens der thermisch expandierbaren Plastisole (gemäß Beispiel 10) enthaltend die erfindungsgemäßen Furandicarbonsäureestern und Diisononyl(ortho)phthalat bzw. Schnellgelierer bei 200 °C.

Das Aufschäumverhalten wurde mit Hilfe eines Dickenschnellmessers (mit Eignung für Weich-PVC Messungen) mit einer Genauigkeit von 0,01 mm bestimmt. Für die Folienherstellung wurde am Rollrakel eines Mathis Labcoaters (Hersteller: Fa. W. Mathis AG) ein Rakelspalt von 1 mm eingestellt. Dieser wurde mit einer Fühlerblattlehre kontrolliert und gegebenenfalls nachgestellt. Die in Beispiel 10 hergestellten Plastisole wurden auf ein in einem Rahmen plan eingespanntes Trennpapier (Warran Release Paper; Fa. Sappi Ltd.) mittels dem Rollrakel des Mathis Labcoaters aufgerakelt. Um die prozentuale Aufschäumung errechnen zu können, wurde zunächst eine angelierte und nicht geschäumte Folie hergestellt. Die Foliendicke dieser Folie betrug bei dem angegebenen Rakelspalt 0,74 mm. Die Messung der Dicke wurde an drei unterschiedlichen Stellen der Folie durchgeführt.

Anschließend wurden ebenfalls mit dem bzw. im Mathis-Labcoater die geschäumten Folien (Schäume) bei 4 unterschiedlichen Ofen-Verweilzeiten (60s, 90s, 120s und 150s) hergestellt. Nach Abkühlung der Schäume wurden die Dicken ebenfalls an drei unterschiedlichen Stellen vermessen. Der Mittelwert der Dicken und die Ausgangsdicke von 0,74 mm wurden für die Berechnung der Expansion benötigt. (Beispiel: (Schaumdicke-Ausgangsdicke)/Ausgangsdicke*100 % = Expansion). Die Ergebnisse werden in der nachfolgenden Tabelle (10) dargestellt.

**Tabelle 10: Expansionsraten der aus den thermisch expandierbaren Plastisolen (gemäß Bsp. 10) bei unterschiedlichen Ofen-Verweilzeiten im Mathis Labcoater bei 200 °C hergestellten Polymerschäume.**

| **Plastisolrezeptur (gemäß Bsp. 10)** | **1**** | **2*** | **3*** | **4*** | **5*** | **6*** |
|---|---|---|---|---|---|---|
| Expansion nach 60 s [%] | 19 | 3 | 0 | 42 | 35 | 28 |
| Expansion nach 90 s [%] | 386 | 386 | 400 | 427 | 386 | 400 |
| Expansion nach 120 s [%] | 454 | 474 | 481 | 501 | 474 | 508 |
| Expansion nach 150 s [%] | 488 | 508 | 515 | 522 | 495 | 528 |

| | | | | | | |
|---|---|---|---|---|---|---|
| ** = Vergleichsbeispiel * = Erfindungsgemäß | | | | | | |

Die Expansion der erfindungsgemäßen Plastisolrezepturen, welche Furandicarbonsäurediisononylester enthalten, verläuft deutlich schneller als mit dem im Vergleichsbeispiel (Plastisolrezeptur 1) alleine verwendeten Diisononylphthalat als Weichmacher. Durch die gezielte Verwendung bestimmter Schnellgelierer wie z. B. bestimmter Citronensäureester (Plastisolrezeptur 3) lassen sich PVC-Plastisole herstellen, die einerseits einer thermischen Vorbehandlung (z. B. Vorgelierung bei Mehrschichtaufbau) unterzogen werden können, ohne dabei schon eine messbare Expansion aufzuweisen, andererseits aber im folgenden um so schneller Expandieren. Durch die Auswahl anderer Weichmacher-Kombinationspartner lassen sich ebenso PVC-Plastisole herstellen, welche (wie z. B. Plastisolrezepturen 4 und 5) gleich zu Beginn eine starke Expansion aufweisen, und so eine im Vergleich zum derzeitigen Standardweichmacher DINP bedeutend kürzere Gesamtverarbeitungszeit erlauben. Es werden somit PVC-Plastisole zur Verfügung gestellt, die eine große Bandbreite unterschiedlicher Verarbeitungsmöglichkeiten aufweisen.

### Beispiel 13:

### Bestimmung des Gelierverhaltens von thermisch expandierbaren Plastisolen (gemäß Beispiel 10) enthaltend die erfindungsgemäßen Furandicarbonsäureestern und Diisononyl(ortho)phthalat bzw. Schnellgelierer.

Die Untersuchung des Gelierverhaltens der in Beispiel 10 hergestellten thermisch expandierbaren Plastisole wurde im Physica MCR 101 im Oszillationsmodus mit einem Platte-Platte Meßsystem (PP25), welches schubspannungsgesteuert betrieben wurde, gemäß der in Beispiel 6 beschriebenen Vorgehensweise vorgenommen.

Bestimmt wurde die sog. komplexe Viskosität des Plastisols in Abhängigkeit von der Temperatur bei konstanter Heizrate (sog. Gelierkurve). Ein Einsetzen des Geliervorganges ist in einem plötzlichen starken Anstieg der komplexen Viskosität zu erkennen. Je früher dieser Viskositätsanstieg einsetzt, desto schneller geliert das entsprechende Plastisol. Aus den erhaltenen Meßkurven wurden durch Interpolation für jedes Plastisol die Temperaturen bestimmt, bei der eine komplexe Viskosität von 1000 Pa * s bzw. 10.000 Pa*s erreicht war. Zusätzlich wurde mittels Tangentenmethode die im vorliegenden Versuchsaufbau maximal erreichte Plastisolviskosität bestimmt, sowie durch Fällen eines Lotes die Temperatur, ab der die maximale Plastisolviskosität auftritt. Die Ergebnisse werden in der nachfolgenden Tabelle (11) dargestellt.

**Tabelle 11: Aus den Gelierkurven (Viskositätskurven) bestimmte Eckpunkte des Gelierverhaltens der gemäß Beispiel 10 hergestellten thermisch expandierbaren Plastisole.**

| **Plastisolrezeptur (gemäß Bsp. 10)** | **1**** | **2*** | **3*** | **4*** | **5*** | **6*** |
|---|---|---|---|---|---|---|
| Erreichen einer Plastisolviskosität von 1 000 Pa*s bei [°C] | 80 | 80 | 78 | 78 | 77 | 74 |
| Erreichen einer Plastisolviskosität von 10 000 Pa*s bei [°C] | 84 | 84 | 82 | 82 | 80 | 77 |
| Maximale Plastisolviskosität [Pa*s] | 42000 | 41000 | 52500 | 53000 | 43000 | 61000 |
| Temperatur beim Erreichen der maximalen Plastisolviskosität [°C] | 92 | 90 | 88 | 88 | 88 | 86 |

| | | | | | | |
|---|---|---|---|---|---|---|
| ** = Vergleichsbeispiel * = Erfindungsgemäß | | | | | | |

Durch die Wahl des Schnellgelierers ist nicht nur - wie zu erwarten - die Geliergeschwindigkeit sowie die Geliertemperatur einstellbar, sondern in einem überraschend hohen Ausmaß auch die maximale Viskosität des ausgelierten Plastisols (maximale Plastisolviskosität), und somit die Materialeigenschaften des durch die thermische Expansion hergestellten PVC-Schaumes. Es werden somit thermisch expandierbare PVC-Plastisole zur Verfügung gestellt, die einerseits wesentlich schneller gelieren als Plastisole, die mit dem derzeitigen Standardweichmacher DINP alleine hergestellt wurden, andererseits aber auch zu Schäumen mit deutlich höherer Viskosität bzw. deutlich höherer Festigkeit und/oder Elastizität verarbeitet werden können.

### Beispiel 14:

### Verwendung der erfindungsgemäßen Furandicarbonsäureester zusammen mit anderen Weichmachern in Dryblends - Herstellung der Dryblends.

Die mit den erfindungsgemäßen Estern erzielbaren vorteilhaften Eigenschaften sollen im Folgenden exemplarisch an Trockenmischungen, sogenannte "Dryblends", und den aus diesen erhältlichen Halbzeugen aufgezeigt werden. Die hergestellten Rezepturen sind in der nachfolgenden Tabelle (12) dargestellt.

**Tabelle 12: Rezepturen der Trockenmischungen**

| [Alle Angaben in phr (= Massenteile pro 100 Massenteile PVC)] | | | | | | | |
|---|---|---|---|---|---|---|---|
| **Trockenmischung** | **1**** | **2*** | **3*** | **4*** | **5*** | **6*** | **7*** |
| Solvin S 271 PC | 100 | 100 | 100 | 100 | 100 | 100 | 100 |
| Vestinol 9 | 50 | 37,5 | | | | | |
| DEHT | | | 37,5 | | | | |
| DINT | | | | 37,5 | | | |
| DINCH | | | | | 37,5 | | |
| GSS | | | | | | 37,5 | |
| Polysorb ID 37 | | | | | | | 37,5 |
| DINFDC | | 12,5 | 12,5 | 12,5 | 12,5 | 12,5 | 12,5 |
| Drapex 39 | 3 | 3 | 3 | 3 | 3 | 3 | 3 |
| Mark BZ 561 | 2 | 2 | 2 | 2 | 2 | 2 | 2 |
| Calciumstearat | 0,4 | 0,4 | 0,4 | 0,4 | 0,4 | 0,4 | 0,4 |

| | | | | | | | |
|---|---|---|---|---|---|---|---|
| ** = Vergleichsbeispiel * = Erfindungsgemäß | | | | | | | |

Die verwendeten Stoffe und Substanzen werden im Folgenden näher erläutert:
**Solvin S 271 PC:** Suspensions-PVC mit einem K-Wert (Bestimmt nach DIN EN ISO 1628-2) von 71; Fa. SOLVIN S.A.
**Vestinol 9:** Diisononyl(ortho)phthalat [DINP], Weichmacher; Fa. Evonik Oxeno GmbH.
**DEHT:** Di(2-ethylhexyl)terephthalat; "Eastman 168"; Weichmacher; Fa. Eastman Chemical.
**DINT:** Diisononylterephthalat (Laborprodukt, Herstellung gemäß DE 102008006400A1 / Beispiel 1)
**DINCH:** Di(isononyl)cyclohexandicarbonsäurediester; Hexamoll DINCH; Weichmacher; Fa. BASF AG.
**GSS:** Octadecansäure-12-(acetyloxy)-2,3-bis(acetyloxy)propylester; Glycerintriester hergestellt auf Basis von Ricinusöl; "Grindstedt Soft'n Safe"; Weichmacher; Fa. Danisco A/S.
Polysorb ID 37: Isosorbid-di(octansäure)ester; Weichmacher; Fa. Roquette Freres.
**DINFDC:** erfindungsgemäßer Furan-2,5-dicarbonsäurediisononylester; Herstellung gemäß Beispiel 2.
**Drapex 39:** Epoxidiertes Sojabohnenöl; Costabilisator & Co-Weichmacher; Fa. Chemtura / Galata.
**Mark BZ 561:** Barium/Zink-Stabilisator; Fa. Chemtura / Galata.
**Calciumstearat:** Calciumsalz der Stearinsäure; Gleitmittel.

Die Herstellung der Dryblends erfolgte in einem Brabender Planetenmischer. Ein Thermostat erhitzte den Mischbehälter des Planetenmischers auf eine konstante Temperatur von 90 °C. Über die Software "Winmix" wurden folgende Parameter am Brabender Planetenmischer eingestellt.
Drehzahlprogramm: Aktiv
Profil: Drehzahl 50 U/min; Haltezeit: 9 min; Anstiegszeit: 1 min Drehzahl 100 U/min; Haltezeit: 20 min
Knetertemperatur: 88 °C
Messbereich: 2 Nm
Dämpfung: 3

Die Temperatur im Mischbehälter betrug 88 °C. Nachdem der Planetenmischer eine Eigenkalibrierung durchgeführt hatte, wurden die festen Bestandteile dem Mischgefäß zugeführt. Das Programm wurde gestartet und die Pulvermischung wurde 10 Minuten im Mischgefäß gerührt und temperiert, ehe die flüssigen Bestandteile zugegeben wurden. Die Mischung wurde weitere 20 Minuten im Planetenmischer gerührt. Nach Beendigung des Programms wurde die fertige Trockenmischung (Pulver) entnommen. Das übertragende Drehmoment-Zeit Diagramm wurde über die BRABENDER-Software ausgewertet. Nach der Zugabe der flüssigen Bestandteile ist ein deutlicher Kurvenanstieg zu erkennen. Erst wenn die Kurve wieder deutlich abfällt, ist die Weichmacheraufnahme abgeschlossen. Die Zeitdifferenz dieser beiden Punkte ist die Weichmacheraufnahmezeit (sog. Dryblendzeit). Das maximale Drehmoment wird vom Programm automatisch ausgewertet. Die Weichmacheraufnahme sowie das bei der Herstellung der Trockenmischungen bestimmte maximale Drehmoment sind in Tabelle 13 dargestellt.

**Tabelle 13: Für die Aufnahme der flüssigen Rezepturkomponenten durch das vortemperierte PVC benötigte Zeit (Weichmacheraufnahme) und das bei der Herstellung der Trockenmischungen bestimmte maximale Drehmoment.**

| Trockenmischung gemäß Bsp. 14 | 1** | 2* | 3* | 4* | 5* | 6* | 7* |
|---|---|---|---|---|---|---|---|
| Weichmacheraufnahme [min.] | 4,5 | 4,2 | 4,8 | 6,2 | 5,5 | 3,7 | 3,3 |
| Maximales Drehmoment [Nm] | 1,1 | 1,1 | 1 | 1,1 | 1,1 | 1,1 | 1 |

| | | | | | | | |
|---|---|---|---|---|---|---|---|
| ** = Vergleichsbeispiel * = Erfindungsgemäß | | | | | | | |

Die Verarbeitungsgeschwindigkeit der erfindungsgemäßen Mischungen liegt teilweise deutlich höher als die der Vergleichsrezeptur mit dem Standardweichmacher DINP, dabei ist das maximale Drehmoment in allen Fällen vergleichbar. Es werden somit Trockenmischungen / Dryblends zur Verfügung gestellt, die gegenüber dem bisherigen Standardweichmacher DINP eine deutlich höhere Verarbeitungsgeschwindigkeit bei ähnlicher Kraftaufnahme erlauben.

### Beispiel 15:

### Herstellung von Walzfellen und Preßplatten aus den die erfindungsgemäßen Furandicarbonsäureester zusammen mit anderen Weichmachern enthaltenden Trockenmischungen / Dryblends (aus Beispiel 14)

### Herstellung der Walzfelle

Die Herstellung der Walzfelle erfolgte auf einem Kalander W150 AP der Fa. Collin. Folgende Parameter wurden am Kalander eingestellt:
Walzentemperatur: 165 °C
Walzenspalt: 0,5 mm
Walzzeit: 5 min
Fünfstufiges Programm zur Herstellung des Walzfelles

Nach dem Erreichen der Walzentemperatur wurde der Walzenspalt kalibriert. Zum Start der Messung wurde der Walzenspalt auf 0,2 mm eingestellt. Jeweils 160 Gramm eines Dryblends (aus Beispiel 14) wurden eingewogen und bei stehenden Walzen in den Walzenspalt gegeben. Das Programm wurde gestartet. Die Walzen starteten mit einer Umdrehungszahl von 5 U/min und einer Friktion von 20 %. Nach ca. 1 min war die Plastifizierung zum größten Teil abgeschlossen und der Walzenspalt wurde auf 0,5 mm vergrößert. Es erfolgte eine 3malige Homogenisierung mittels automatischer Umlegeeinheit am Kalander. Nach 5 min wurde das Walzfell von der Walze entfernt und abgekühlt.

### Herstellung der Pressplatten

Die Pressplatten wurden an einer Laborpresse der Fa. Collin hergestellt. Die vorgefertigten Walzfelle (siehe oben) wurden zur Herstellung der Pressplatten verwendet. Die Seitenränder der Walzfelle wurden mit Hilfe einer Schneidemaschine entfernt, das Walzfell wurde anschließend in ca. 14,5 x 14,5 cm große Stücke geschnitten. Für 1 mm dicke Pressplatten wurden je 2 Walzfellstücke in den 15 x 15 cm großen Pressrahmen aus Edelstahl gelegt.

Folgende Parameter wurden an der Laborpresse eingestellt:
Dreiphasiges Programm:
   Phase 1: Beide Platten 165°; Pressplattendruck: 5 bar; Phasenzeit: 60 Sekunden.
   Phase 2: Beide Platten 165°; Pressplattendruck: 200 bar; Phasenzeit: 120 Sekunden.
   Phase 3: Beide Platten 40°; Pressplattendruck: 200 bar; Phasenzeit: 270 Sekunden.

Die überschüssige Presslippe wurde nach Herstellung der Pressplatten entfernt.

### Beispiel 16:

### Bestimmung der weichmachenden Wirkung bzw. der Weichmachereffizienz an Preßplatten hergestellt aus den die erfindungsgemäßen Furandicarbonsäureester zusammen mit anderen Weichmachern enthaltenden Trockenmischungen / Dryblends durch Bestimmung der Shore-Härte (Shore A und D)

Die Shore-Härte ist ein Maß für die Weichheit eines Probekörpers. Je weiter bei einer bestimmten Messdauer eine genormte Nadel in den Probenkörper eindringen kann, desto niedriger fällt der Messwert aus. Der Weichmacher mit der höchsten Effizienz ergibt bei gleicher Weichmachermenge den niedrigsten Wert für die Shore Härte. Da in der Praxis Formulierungen / Rezepturen häufig auf eine bestimmte Shorehärte hin eingestellt bzw. optimiert werden, kann bei sehr effizienten Weichmachern demnach ein bestimmter Anteil in der Rezeptur eingespart werden, was eine Kostenreduktion für den Verarbeiter bedeutet.

Die Härte-Messungen wurden nach DIN 53 505 mit einem Shore-A- und einem Shore-D-Messgerät der Fa. Zwick-Roell durchgeführt, der Messwert jeweils nach 3 Sekunden abgelesen. An jedem Probekörper (hergestellt gemäß Beispiel 15) wurden Messungen an drei verschiedenen Stellen durchgeführt, und ein Mittelwert gebildet. Die Ergebnisse der Härtebestimmung sind in Tabelle 14 zusammengestellt.

**Tabelle 14: Härte nach Shore A und Shore D an Preßplatten hergestellt (gemäß Beispiel 15) aus den die erfindungsgemäßen Furandicarbonsäureester zusammen mit anderen Weichmachern enthaltenden Trockenmischungen / Dryblends (gemäß Beispiel 14).**

| Trockenmischung gemäß Bsp. 14 | 1** | 2* | 3* | 4* | 5* | 6* | 7* |
|---|---|---|---|---|---|---|---|
| Shore A | 85 | 85 | 86 | 88 | 86 | 83 | 85 |
| Shore D | 28 | 28 | 28 | 30 | 28 | 26 | 27 |

| | | | | | | | |
|---|---|---|---|---|---|---|---|
| ** = Vergleichsbeispiel * = Erfindungsgemäß | | | | | | | |

Durch die Abmischung von unterschiedlichen Standardweichmachern mit den erfindungsgemäßen Furandicarbonsäureestern in Dryblends wird eine Weichmachereffizienz erzielt, die ähnlich der des DINP (Standardweichmacher) oder besser ist. Es werden dabei auch Trockenmischungen / Dryblends zur Verfügung gestellt, die gegenüber dem derzeit als Universalweichmacher eingesetzten DINP eine deutlich verbesserte Effizienz aufweisen und daher insbesondere zu niedrigeren Rezepturkosten führen können.

### Beispiel 17:

### Bestimmung der Wasseraufnahme und des Auswaschverhaltens an Preßplatten (Halbzeugen) hergestellt aus den die erfindungsgemäßen Furandicarbonsäureester zusammen mit anderen Weichmachern enthaltenden Trockenmischungen / Dryblends

Wasseraufnahme und Auswaschverhalten sind zwei wesentliche Kriterien bei der Beurteilung der Güte von Halbzeugen welche auf Basis von PVC-Dryblends hergestellt wurden. Nimmt ein PVC-Halbzeug in größerem Umfang Wasser auf, so verändern sich dadurch einerseits seine Materialeigenschaften, andererseits auch sein optisches Aussehen (z. B. Eintrübung). Eine hohe Wasseraufnahme ist demnach in der Regel unerwünscht. Das Auswaschverhalten ist ein zusätzliches Kriterium für die Permanenz der Formulierungsbestandteile unter Gebrauchsbedingungen (z. B. bei Fußbodenbelägen oder Dachbahnen). Dies gilt insbesondere für Stabilisatoren, Weichmacher und/oder ihre Bestandteile, da eine Konzentrationsminderung im Halbzeug bei diesen Rezepturbestandteilen sowohl die Materialeigenschaften verschlechtern als auch die Lebensdauer der Halbzeuge dramatisch reduzieren kann.

### Herstellung der Prüfkörper

Aus den Pressplatten (hergestellt gemäß Beispiel 15) wurden pro Probe/Trockenmischung jeweils 3 Kreise (a 10cm²) mit Hilfe eines Kreisschneiders ausgeschnitten. Die Kreise wurden gelocht. Vor der Wasserlagerung wurden die Kreise für 24 Stunden in einem mit Trockenmittel (KC-Trockenperlen) ausgerüsteten Exsikkator bei 25 °C gelagert. Das Ausgangsgewicht (Einwage) wurde mit einer Analysenwaage auf 0,1 mg genau bestimmt. Die Kreise wurden nun in einem mit VE-Wasser gefülltem Schüttelbad bei einer Temperatur von 30°C für 24 Stunden mit geeigneten Probenhaltern unter der Wasseroberfläche gelagert und kontinuierlich bewegt. Nach der Lagerung wurden die Kreise dem Wasserbad entnommen, abgetrocknet und ausgewogen (Gewicht nach 24h). Die ausgewogenen Kreise wurden erneut im Wasserbad platziert und nach 7 Tagen erneut im abgetrockneten Zustand ausgewogen (Gewicht nach 7 Tagen). Nach der zweiten Auswage wurden die Kreise wiederum für 24 Stunden in einem mit Trockenmittel (KC-Trockenperlen) ausgerüsteten Exsikkator bei 25 °C gelagert und anschließend nochmals ausgewogen (Endauswage = Gewicht nach Trocknung). Die Gewichtsänderungen wurden prozentual errechnet und sind in Tabelle 15 dargestellt.

**Tabelle 15: Wasseraufnahme und Auswaschverhaltens bestimmt an Prüfkörpern hergestellt aus Preßplatten (gemäß Beispiel 15) welche aus den die erfindungsgemäßen Furandicarbonsäureester zusammen mit anderen Weichmachern enthaltenden Trockenmischungen / Dryblends (gemäß Beispiel 14) hergestellt wurden.**

| Trockenmischung gemäß Bsp. 14 | 1** | 2* | 3* | 4* | 5* | 6* | 7* |
|---|---|---|---|---|---|---|---|
| Gewichtsänderung nach 1 Tag [ma%] | + 0,26 | + 0,27 | + 0,24 | + 0,24 | + 0,3 | +0,4 | + 0,06 |
| Gewichtsänderung nach 7 Tagen [ma%] | + 0,33 | + 0,34 | + 0,34 | + 0,38 | + 0,41 | + 0,35 | - 0,57 |
| Gewichtsänderung nach Trocknung [ma%] | - 0,16 | - 0,14 | - 0,17 | - 0,13 | - 0,16 | - 0,35 | - 1,16 |

| | | | | | | | |
|---|---|---|---|---|---|---|---|
| ** = Vergleichsbeispiel * = Erfindungsgemäß | | | | | | | |

Die Prüfkörper, welche den erfindungsgemäßen Furandicarbonsäureester enthalten, verhalten sich hinsichtlich der Wasserlagerung analog zum Prüfkörper, welcher allein den Standardweichmacher DINP enthält. Die Wasseraufnahme ist äußerst gering, was insbesondere für kalandrierte Fußbodenbeläge aber auch für Dachbahnen von Vorteil ist. Auch der Masseverlust durch Auswaschung hält sich mit Ausnahme der Mischung von Isosorbidester Polysorb ID 37 und Furandicarbonsäurediisononylester in engen Grenzen. Es werden somit Dryblends und daraus herstellbare Halbzeuge zur Verfügung gestellt, die sich durch niedrige Wasseraufnahme und geringes Auswaschverhalten auszeichnen und somit ideal auch für den Einsatz in Bereichen mit ständigem oder häufigem Wasserkontakt geeignet sind.

### Beispiel 18:

### Bestimmung der Zug-/Dehnungseigenschaften an Preßplatten (Halbzeugen) hergestellt aus den die erfindungsgemäßen Furandicarbonsäureester zusammen mit anderen Weichmachern enthaltenden Trockenmischungen / Dryblends.

Reißfestigkeit und Reißdehnung sind Materialeigenschaften, die insbesondere für mechanisch belastete Halbzeuge eine wichtige Rolle spielen. Die mechanische Belastung kann dabei sowohl während des Herstellprozesses des Halbzeuges als auch während seines Gebrauchs auftreten. Bevorzugt (insbesondere im Bereich Dachbahn) werden in den meisten Fällen Materialien, die eine hohe Reißfestigkeit bei moderater Dehnung aufweisen.

Für die Zugprüfungen wurden aus den gemäß Beispiel 15 hergestellten Preßplatten normgerechte "S-2"-Prüfstäbe ausgestanzt. Die Zugprüfungen erfolgten nach DIN 53504 an einem Zugprüfgerät "Z 1445" der Fa. Zwick.

Folgende Prüfbedingungen wurden eingestellt:
Prüfklima: 23 °C, 50 %rF
Vorkraft: 0,5 N
Geschwindigkeit Vorkraft: 5 mm/min.
Prüfgeschwindigkeit: 100 mm/min.

Für die Bestimmung der Reißfestigkeit und der Reißdehnung wurden 5 Messungen je Probe durchgeführt. Die gemittelten Messwerte wurden in die nachfolgende Tabelle (16) eingetragen.

**Tabelle 16: Zugeigenschaften bestimmt nach DIN 53504 an S2-Prüfkörpern, hergestellt aus Preßplatten (gemäß Beispiel 15) welche aus den die erfindungsgemäßen Furandicarbonsäureester zusammen mit anderen Weichmachern enthaltenden Trockenmischungen / Dryblends (gemäß Beispiel 14) hergestellt wurden.**

| Trockenmischung gemäß Bsp. 14 | 1** | 2* | 3* | 4* | 5* | 6* | 7* |
|---|---|---|---|---|---|---|---|
| Reißfestigkeit [MPa] | 23,7 | 23,9 | 25,3 | 25,6 | 23,8 | 25,0 | 26,6 |
| Reißdehnung [%] | 285 | 300 | 305 | 300 | 290 | 325 | 320 |

| | | | | | | | |
|---|---|---|---|---|---|---|---|
| ** = Vergleichsbeispiel * = Erfindungsgemäß | | | | | | | |

Durch die Verwendung der erfindungsgemäßen Mischungen lässt sich im Vergleich zum reinen DINP (Standardweichmacher) eine erhebliche Erhöhung der Reißfestigkeit erzielen. Dabei wird die Materialflexibilität in keiner Weise eingeschränkt sondern die Reißdehnung sogar leicht erhöht. Es werden somit Dryblends und daraus herstellbare Halbzeuge zur Verfügung gestellt, die sich durch eine hohe Reißfestigkeit bei gleichzeitig hoher Flexibilität und guter Formbeständigkeit auszeichnen, und sich daher auch für den Einsatz bei hoher mechanischer Belastung (u. a. zum Verspannen) eignen.

### Beispiel 19:

### Verwendung der erfindungsgemäßen Furandicarbonsäureester in Schutzmassen (z. B. Unterbodenschutz / UBS) - Herstellung der UBS-Plastisole

Die mit den erfindungsgemäßen Estern erzielbaren vorteilhaften Eigenschaften sollen im Folgenden an UBS-Plastisolen (Schutzmassen) aufgezeigt werden. Die Herstellung der Plastisole erfolgte gemäß Beispiel 4 jedoch mit veränderter Rezeptur. Die verwendeten Einwaagen der Komponenten für die verschiedenen Plastisole sind der nachfolgenden Tabelle (17) zu entnehmen.

**Tabelle 17: Rezepturen der UBS-Schutzmassen (Plastisolen)**

| [Alle Angaben in phr (= Massenteile pro 100 Massenteile PVC)] | | |
|---|---|---|
| **Plastisolrezeptur** | **1*** | **2**** |
| Emulsions PVC mit K-Wert 70 (VESTOLIT E 7031, Fa. Vestolit) | 100 | 100 |
| Furan-2,5-dicarbonsäurediisononylester (erfindungsgemäß) | 130 | |
| DINP (VESTINOL 9 der Evonik Oxeno GmbH, Vergleichsbeispiel) | | 130 |
| Beschichtetes Calciumcarbonat (Socal 312, Fa. Solvay Chemicals) | 70 | 70 |
| Weißkalk DIN EN 459-1 / CL-Q-Feinkalk (Precal 30 S, Fa. Schäfer-Krusemark) | 5 | 5 |
| Haftvermittler (Nouribond 323, Fa. Air Products) | 3 | 3 |
| Zinkoxid (Zinkoxid Aktiv; Fa. Lanxess AG) | 1 | 1 |
| Aliphatisches Lösungsmittel (Shellsol D70; Fa. Shell Chemicals) | 5 | 5 |

| | | |
|---|---|---|
| ** = Vergleichsbeispiel * = Erfindungsgemäß | | |

### Beispiel 20:

### Bestimmung der Plastisolviskosität von UBS-Plastisolen enthaltend die erfindungsgemäßen Furandicarbonsäureester nach einer Lagerdauer von 2 h (bei 25 °C)

Schutzmassen, insbesondere solche, die im Bereich Kfz-Unterbodenschutz eingesetzt werden, sollen je nach vorliegender Schergeschwindigkeit unterschiedliche Viskositätsanforderungen erfüllen. So sollen sie während der Applikation, welche in der Regel unter hohen Schergeschwindigkeiten durch Versprühen erfolgt, möglichst leicht fließen und auf der behandelten Oberfläche ein homogenes Sprühbild und einen guten Verlauf aufweisen. Nach der Applikation (d. h. bei weitestgehender Abwesenheit einer Scherkraft) sollen sie demgegenüber eine hohe Viskosität aufweisen und nur ein geringfügiges Nachlaufverhalten zeigen.

Die Messung der Viskositäten der in Beispiel 19 hergestellten Plastisole erfolgte mit einem Rheometer Physica MCR 101 (Fa. Paar-Physica), gemäß der in Beispiel 5 beschriebenen Vorgehensweise nach einer Temperierzeit der Pasten von 2 Stunden bei 25 °C. Die Ergebnisse werden in der nachfolgenden Tabelle (18) exemplarisch für die Schergeschwindigkeiten 100/s, 10/s, 1/s und 0,1/s dargestellt.

**Tabelle 18: Scherviskosität der Plastisole aus Beispiel 19 nach 2h Lagerung bei 25 °C.**

| **Plastisolrezeptur gemäß Bsp. 19** | **1*** | **2**** |
|---|---|---|
| Scherviskosität bei Schergeschwindigkeit= 100/s [Pa*s] | 7,2 | 7,2 |
| Scherviskosität bei Schergeschwindigkeit= 10/s [Pa*s] | 50 | 54,5 |
| Scherviskosität bei Schergeschwindigkeit= 1/s [Pa*s] | 423 | 437 |
| Scherviskosität bei Schergeschwindigkeit= 0,1/s [Pa*s] | 1590 | 1150 |

| | | |
|---|---|---|
| ** = Vergleichsbeispiel * = Erfindungsgemäß | | |

Im Vergleich mit DINP (Standardweichmacher, Plastisolrezeptur 2) weist die auf der erfindungsgemäßen Furandicarbonsäureester-Mischung basierende UBS-Paste / das UBS-Plastisol (Plastisolrezeptur 1) bei hohen Schergeschwindigkeiten eine ebenso niedrige Scherviskosität auf, während sie bei niedrigen Schergeschwindigkeiten deutlich über der Scherviskosität der DINP-Paste liegt. Bei gleicher Verarbeitbarkeit im Auftrag ist damit ein eindeutiger Vorteil bei der Tropffestigkeit der Formulierung gegeben. Es werden somit UBS-Plastisole zur Verfügung gestellt, die bei exzellenten Versprüh- und Verlaufseigenschaften gleichzeitig ein nur sehr geringes Nachlaufverhalten zeigen.

### Beispiel 21:

### Bestimmung der Geliergeschwindigkeit von UBS-Plastisolen enthaltend die erfindungsgemäßen Furandicarbonsäureester.

Die Gelierung der UBS-Schutzmassen muss innerhalb der in der Automobilindustrie vorhandenen thermischen Härtungsverfahren möglich sein. Dabei kommt es einerseits auf eine möglichst schnelle Verfestigung der Schutzmasse an, um ein nachträgliches Abtropfen zu unterbinden, andererseits aber auch auf eine möglichst vollständige Gelierung innerhalb möglichst kurzer Zeit, um eine maximale Schutzwirkung zu erreichen. Die Untersuchung des Gelierverhaltens der in Beispiel 19 hergestellten UBS-Plastisole wurde im Physica MCR 101 im Oszillationsmodus mit einem Platte-Platte Meßsystem (PP25), welches schubspannungsgesteuert betrieben wurde, gemäß der in Beispiel 6 beschriebenen Vorgehensweise vorgenommen.

Bestimmt wurde die sog. komplexe Viskosität des Plastisols in Abhängigkeit von der Temperatur bei konstanter Heizrate (sog. Gelierkurve). Ein Einsetzen des Geliervorganges ist in einem plötzlichen starken Anstieg der komplexen Viskosität zu erkennen. Je früher dieser Viskositätsanstieg einsetzt, desto schneller geliert das entsprechende Plastisol. Aus den erhaltenen Meßkurven wurden durch Interpolation für jedes Plastisol die Temperaturen bestimmt, bei der eine komplexe Viskosität von 1000 Pa * s bzw. 10.000 Pa*s erreicht war. Zusätzlich wurde mittels Tangentenmethode die im vorliegenden Versuchsaufbau maximal erreichte Plastisolviskosität bestimmt, sowie durch Fällen eines Lotes die Temperatur, ab der die maximale Plastisolviskosität auftritt. Die Ergebnisse werden in der nachfolgenden Tabelle (19) dargestellt.

**Tabelle 19: Aus den Gelierkurven (Viskositätskurven) bestimmte Eckpunkte des Gelierverhaltens der gemäß Beispiel 19 hergestellten UBS-Plastisole.**

| **Plastisolrezeptur gemäß Bsp. 19** | **1*** | **2**** |
|---|---|---|
| Erreichen einer Plastisolviskosität von 1 000 Pa*s bei [°C] | 50 | 74 |
| Erreichen einer Plastisolviskosität von 10 000 Pa*s bei [°C] | 75 | 110 |
| Maximale Plastisolviskosität [Pa*s] | 82 000 | 13 000 |
| Temperatur beim Erreichen der maximalen Plastisolviskosität [°C] | 124 | 130 |

| | | |
|---|---|---|
| ** = Vergleichsbeispiel * = Erfindungsgemäß | | |

Die UBS-Schutzmasse, welche die erfindungsgemäßen Furandicarbonsäureester enthält, zeigt eine wesentlich schnellere Gelierung als die vergleichbare DINP-Schutzmasse, d.h. dass eine wesentlich schnellere Verarbeitung oder alternativ dazu die Verwendung von niedrigeren Verarbeitungstemperaturen (= Energie- und Kosteneinsparung) möglich ist. Die wesentlich höhere Endviskosität der erfindungsgemäßen UBS-Schutzmasse weist außerdem auf eine verbesserte Schutzwirkung der Masse z. B. gegen Steinschlag hin. Es werden somit UBS-Plastisole zur Verfügung gestellt, die im Vergleich mit UBS-Plastisolen, welche auf dem derzeitigen Standardweichmacher DINP basieren bedeutend bessere Verarbeitung- und Materialeigenschaften aufweisen.

### Beispiel 22:

### Haftungswirkung von UBS-Schutzmassen enthaltend die erfindungsgemäßen Furandicarbonsäureester auf Standard-Blechen.

Entscheidend für eine anhaltende Schutzwirkung von UBS-Schutzmassen sind u.a. die Haftungseigenschaften der ausgelierten UBS-Schutzmassen auf den Kfz-Karosserieblechen.

Für die Haftungsprüfung wurden speziell beschichtete "Cathogard" Bleche (BASF Coatings GmbH) benutzt. Mit Hilfe eines Rakels wurden die Bleche mit den UBS-Plastisolen aus Beispiel 19 beschichtet. Eingesetzt wurden die UBS-Plastisole nach einer Lagerdauer von 2 h bei 25 °C. Für die Beschichtung wurden die Bleche mit Klebeband so abgeklebt, dass vier ca. 7 * 3 cm große Felder entstanden. Die Plastisole wurden mit einem Spatel zuerst auf die vier Felder verteilt. Mit dem Rakel wurden die Plastisole dann glatt gestrichen. Überschüssiges Plastisol und das Klebeband wurde entfernt. Die beschichteten Bleche wurden bei einer Temperatur von 130 °C im Trockenschrank für 25 min. ausgeliert.

Die Haftungsprüfung wurde nach drei unterschiedlichen Zeitvorgaben (2 Stunden / 24 Stunden / 168 Stunden) durchgeführt. Dafür wurden die Felder mit einer Rasierklinge in mehrere kleine Felder geteilt. Anschließend wurde mit einem Spezialspatel versucht das erste Feld abzulösen. Das Haftungs-/Ablöseverhalten wurde bewertet (siehe Tabelle 20). Die Lagerung der ausgelierten Bleche zwischen den Prüfungen fand bei 25°C im Temperierschrank statt.

**Tabelle 20: Bewertungssystem für die Haftungs-/Ablöseprüfung der ausgelierten UBS-Plastisole.**

| Bewertung | Bedeutung |
|---|---|
| 1 | Sehr gute Haftung |
| 2 | Gute Haftung |
| 3 | Befriedigende Haftung |
| 4 | Ausreichende Haftung |
| 5 | Mangelhafte Haftung |
| 6 | Ungenügende / Keine Haftung |

Die Ergebnisse der Haftungs-/Ablöseprüfung der ausgelierten UBS-Plastisole sind in der nachfolgenden Tabelle (21) zusammengestellt.

**Tabelle 21: Haftungs-/Ablöseeigenschaften der ausgelierten UBS-Plastisole, hergestellt gemäß Beispiel 19.**

| **Plastisolrezeptur gemäß Bsp. 19** | **1*** | **2**** |
|---|---|---|
| Haftung nach 2h Lagerung der ausgelierten Probe | 3 | 3 |
| Haftung nach 24 h Lagerung der ausgelierten Probe | 3 | 3 |
| Haftung nach 168 h Lagerung der ausgelierten Probe | 3 | 3 |

| | | |
|---|---|---|
| ** = Vergleichsbeispiel * = Erfindungsgemäß | | |

Die die erfindungsgemäßen Furandicarbonsäureester enthaltende Schutzmasse weist somit die gleichen Haftungseigenschaften wie die analoge DINP-Schutzmasse auf. Es werden somit UBS-Schutzmassen zur Verfügung gestellt, die neben sehr guten Verarbeitungs- und Materialeigenschaften auch eine gute Haftung auf Kfz-Blechen und damit eine gute Schutzwirkung aufweisen.

## Patentansprüche

1. Gemische isomerer Nonylester der Furan-2,5-dicarbonsäure der Formel **I**

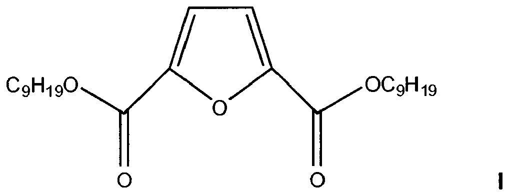

2. Gemisch isomerer Nonylester der Furan-2,5-dicarbonsäure nach Anspruch 1, **dadurch gekennzeichnet, dass** das Gemisch mindestens zwei unterschiedliche Ester enthält, die sich in der Konstitution der isomeren C9-Reste unterscheiden, wobei keiner der im Gemisch vorhandenen C9-Reste einen Anteil von mehr als 90 Mol-%, aufweist.

3. Verfahren zur Herstellung von Gemischen isomerer Nonylester der Furan-2,5-dicarbonsäure der Formel **I** nach Anspruch 1, **dadurch gekennzeichnet, dass**:
a) Furan-2,5-dicarbonsäure in Kontakt gebracht wird mit einem Gemisch isomerer C9-Alkohole unter Freisetzung von Wasser;
b) wobei ein bis zu 50 % molarer Überschuss des Gemisches isomerer C9-Alkohole Verwendung findet;
c) die Umsetzung gemäß a) unter Verwendung eines Katalysators erfolgt, ausgewählt aus den Gruppen der Brønstedt- und/oder der Lewissäuren.

4. Verfahren zur Herstellung von Gemischen isomerer Nonylester der Furan-2,5-dicarbonsäure der Formel **I** nach Anspruch 1, **dadurch gekennzeichnet, dass**
a) Furan-2,5-dicarbonsäure in das entsprechende Furan-2,5-dicarbonsäurechlorid überführt wird, welches
b) nach Abtrennung und Reinigung anschließend in Kontakt gebracht wird mit einem Gemisch isomerer C9-Alkohole unter Freisetzung von Chlorwasserstoff.

5. Verfahren zur Herstellung von Gemischen isomerer Nonylester der Furan-2,5-dicarbonsäure der Formel **I** nach Anspruch 1, **dadurch gekennzeichnet, dass**
a) Furan-2,5-dicarbonsäuredimethylester in Kontakt gebracht wird mit einem Gemisch isomerer C9-Alkohole unter Freisetzung von Methanol;
b) wobei die Umsetzung gemäß a) unter Verwendung eines Katalysators erfolgt, ausgewählt aus den Gruppen der Brønstedt- und/oder der Lewissäuren.

6. Zusammensetzung, enthaltend Gemische isomerer Nonylester der Furan-2,5-dicarbonsäure der Formel **I** nach Anspruch 1 sowie Weichmacher, ausgewählt aus der Gruppe der Alkylbenzoate, der Dialkyladipate, der Glycerinester, der Citronensäuretrialkylester, der acylierten Citronensäuretrialkylester, der Trialkyltrimellitate, der Glykoldibenzoate, der Dialkylterephthalate, der Dialkylphthalate, den Dialkanoylestern des Isosorbid und/oder der Dialkylester der 1,2-, 1,3- oder 1,4-Cyclohexandicarbonsäuren.

7. Zusammensetzung nach Anspruch 6, **dadurch gekennzeichnet, dass** das Verhältnis der isomeren Nonylester der Furan-2,5-dicarbonsäure der Formel I zu den Weichmachern in einem Bereich von 1 zu 15 bis 15 zu 1 liegt.

8. Zusammensetzung nach Anspruch 7, **dadurch gekennzeichnet, dass** sie ein Polymer, ausgewählt aus Polyvinylchlorid, Polyvinylbutyral, Polymilchsäure, Polyhydroxybutyral und/oder Polyalkylmethacrylat, aufweist.

9. Zusammensetzung, enthaltend Gemische isomerer Nonylester der Furan-2,5-dicarbonsäure der Formel **I** nach Anspruch 1 und ein Polymer, ausgewählt aus Polyvinylchlorid, Polyvinylbutyral, Polymilchsäure, Polyhydroxybutyral und/oder Polyalkylmethacrylat.

10. Zusammensetzung nach Anspruch 9, **dadurch gekennzeichnet, dass** das Verhältnis von Polymer zu isomeren Nonylestern der Furan-2,5-dicarbonsäure der Formel **I** in einem Bereich von 30 zu 1 bis 1 zu 2,5 liegt.

11. Verwendung der isomeren Nonylester der Furan-2,5-dicarbonsäure der Formel **I** gemäß einem der Ansprüche 1 bis 2 als Weichmacher.

12. Verwendung der Zusammensetzung nach einem der Ansprüche 6 bis 9 bei der Herstellung von Farben, Tinten, Klebstoffen oder Klebstoffkomponenten, Lacken, Plastisolen, Dichtungsmassen als Weichmacher, insbesondere in Kunststoffen oder Kunststoffkomponenten.

13. Verwendung der Zusammensetzung nach einem der Ansprüche 6 bis 9 als Lösemittel bei der Herstellung von Farben, Tinten, Klebstoffen oder Klebstoffkomponenten, Lacken, Plastisolen, Dichtungsmassen.

14. Verwendung der Zusammensetzung nach einem der Ansprüche 6 bis 9 als Schmierölkomponente.

15. Verwendung der Zusammensetzung nach einem der Ansprüche 6 bis 9 als Hilfsmittel bei der Metallverarbeitung.

## Claims

1. Mixtures of isomeric nonyl esters of furan-2,5-dicarboxylic acid of the formula **I**

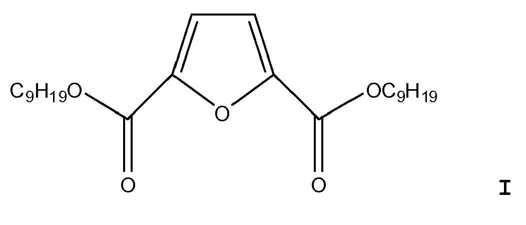

2. Mixture of isomeric nonyl esters of furan-2,5-dicarboxylic acid according to Claim 1, **characterized in that** the mixture comprises at least two different esters which differ in the constitution of the isomeric C9 radicals, with none of the C9 radicals present in the mixture having a fraction of more than 90 mol%.

3. Process for preparing mixtures of isomeric nonyl esters of furan-2,5-dicarboxylic acid of the formula **I** according to Claim 1, **characterized in that**:
a) furan-2,5-dicarboxylic acid is contacted with a mixture of isomeric C9 alcohols, with liberation of water;
b) the mixture of isomeric C9 alcohols is used in a molar excess of up to 50%;
c) the reaction in a) takes place using a catalyst selected from the groups of the Brønsted acids and/or the Lewis acids.

4. Process for preparing mixtures of isomeric nonyl esters of furan-2,5-dicarboxylic acid of the formula **I** according to Claim 1, **characterized in that**
a) furan-2,5-dicarboxylic acid is converted into the corresponding furan-2,5-dicarbonyl chloride which
b) following isolation and purification is subsequently contacted with a mixture of isomeric C9 alcohols, with release of hydrogen chloride.

5. Process for preparing mixtures of isomeric nonyl esters of furan-2,5-dicarboxylic acid of the formula **I** according to Claim 1, **characterized in that**
a) dimethyl furan-2,5-dicarboxylate is brought into contact with a mixture of isomeric C9 alcohols, with release of methanol;
b) the reaction in a) takes place using a catalyst selected from the groups of the Brønsted acids and/or the Lewis acids.

6. Composition comprising mixtures of isomeric nonyl esters of furan-2,5-dicarboxylic acid of the formula **I** according to Claim 1 and also plasticizers selected from the group of the alkyl benzoates, the dialkyl adipates, the glycerol esters, the trialkyl esters of citric acid, the acylated trialkyl esters of citric acid, the trialkyl trimellitates, the glycol dibenzoates, the dialkyl terephthalates, the dialkyl phthalates, the dialkanoyl esters of isosorbide and/or the dialkyl esters of 1,2-, 1,3- or 1,4-cyclohexanedicarboxylic acids.

7. Composition according to Claim 6, **characterized in that** the ratio of the isomeric nonyl esters of furan-2,5-dicarboxylic acid of the formula **I** to the plasticizers is situated in a range from 1:15 to 15:1.

8. Composition according to Claim 7, **characterized in that** it comprises a polymer selected from polyvinyl chloride, polyvinylbutyral, polylactic acid, polyhydroxybutyral and/or polyalkyl methacrylate.

9. Composition comprising mixtures of isomeric nonyl esters of furan-2,5-dicarboxylic acid of the formula **I** according to Claim 1 and a polymer selected from polyvinyl chloride, polyvinylbutyral, polylactic acid, polyhydroxybutyral and/or polyalkyl methacrylate.

10. Composition according to Claim 9, **characterized in that** the ratio of polymer to isomeric nonyl esters of furan-2,5-dicarboxylic acid of the formula **I** is situated in a range from 30:1 to 1:2.5.

11. Use of the isomeric nonyl esters of furan-2,5-dicarboxylic acid of the formula **I** according to either of Claims 1 and 2 as plasticizers.

12. Use of the composition according to any of Claims 6 to 9 in the preparation of paints, inks, adhesives or adhesives components, varnishes, plastisols, sealants as plasticizers, more particularly in plastics or plastics components.

13. Use of the composition according to any of Claims 6 to 9 as solvent in the preparation of paints, inks, adhesives or adhesive components, varnishes, plastisols, sealants.

14. Use of the composition according to any of Claims 6 to 9 as a lubricating oil component.

15. Use of the composition according to any of Claims 6 to 9 as an auxiliary in metals processing.

## Revendications

1. Mélanges d'esters nonyliques isomères de l'acide furanne-2,5-dicarboxylique de formule I

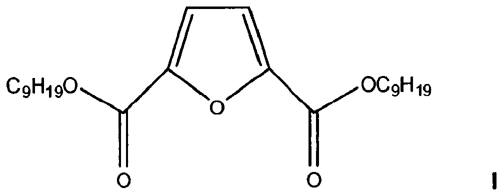

2. Mélanges d'esters nonyliques isomères de l'acide furanne-2,5-dicarboxylique selon la revendication 1, **caractérisés en ce que** le mélange contient au moins deux esters différents, qui se distinguent dans la constitution des radicaux isomères en C₉, aucun des radicaux en C₉ présents dans le mélange ne présentant une proportion supérieure à 90% en mole.

3. Procédé pour la préparation de mélanges d'esters nonyliques isomères de l'acide furanne-2,5-dicarboxylique de formule I selon la revendication 1, **caractérisé en ce que**
a) l'acide furanne-2,5-dicarboxylique est mis en contact avec un mélange d'alcools isomères en C₉ avec libération d'eau ;
b) un excès molaire de jusqu'à 50% du mélange d'alcools isomères en C₉ étant utilisé ;
c) la transformation selon a) ayant lieu avec utilisation d'un catalyseur, choisi parmi les groupes des acides de Brönstedt et/ou de Lewis.

4. Procédé pour la préparation de mélanges d'esters nonyliques isomères de l'acide furanne-2,5-dicarboxylique de formule I selon la revendication 1, **caractérisé en ce que**
a) l'acide furanne-2,5-dicarboxylique est transformé en chlorure de l'acide furanne-2,5-dicarboxylique correspondant, qui
b) est ensuite mis en contact, après séparation et purification, avec un mélange d'alcools isomères en C₉ avec libération de chlorure d'hydrogène.

5. Procédé pour la préparation de mélanges d'esters nonyliques isomères de l'acide furanne-2,5-dicarboxylique de formule I selon la revendication 1, **caractérisé en ce que**
a) de l'ester diméthylique de l'acide furanne-2,5-dicarboxylique est mis en contact avec un mélange d'alcools isomères en C₉ avec libération de méthanol ;
b) la transformation selon a) ayant lieu avec utilisation d'un catalyseur, choisi dans les groupes des acides de Brönstedt et/ou de Lewis.

6. Composition contenant des mélanges d'esters nonyliques isomères de l'acide furanne-2,5-dicarboxylique de formule I selon la revendication 1 ainsi que des plastifiants, choisis dans le groupe des benzoates d'alkyle, des adipates de dialkyle, des esters de glycérol, des esters trialkyliques de l'acide citrique, des esters trialkyliques acylés de l'acide citrique, des trimellitates de trialkyle, des dibenzoates de glycol, des téréphtalates de dialkyle, des phtalates de dialkyle, des esters dialcanoyliques de l'isosorbide et/ou des esters dialkyliques de l'acide 1,2-cyclohexanedicarboxylique, 1,3-cyclohexanedicarboxylique ou 1,4-cyclohexanedicarboxylique.

7. Composition selon la revendication 6, **caractérisée en ce que** le rapport des esters nonyliques isomères de l'acide furanne-2,5-dicarboxylique de formule I aux plastifiants se situe dans une plage de 1:15 à 15:1.

8. Composition selon la revendication 7, **caractérisée en ce qu'**elle présente un polymère, choisi parmi le poly(chlorure de vinyle), le polyvinylbutyral, le poly(acide lactique), le polyhydroxybutyral et/ou le poly((méth)acrylate d'alkyle).

9. Composition, contenant des mélanges d'esters nonyliques isomères de l'acide furanne-2,5-dicarboxylique de formule I selon la revendication 1 et un polymère, choisi parmi le poly(chlorure de vinyle), le polyvinylbutyral, le poly(acide lactique), le polyhydroxybutyral et/ou le poly((méth)acrylate d'alkyle).

10. Composition selon la revendication 9, **caractérisée en ce que** le rapport du polymère aux esters nonyliques isomères de l'acide furanne-2,5-dicarboxylique de formule I se situe dans une plage de 30:1 à 1:2,5.

11. Utilisation des esters nonyliques isomères de l'acide furanne-2,5-dicarboxylique de formule I selon l'une quelconque des revendications 1 à 2 comme plastifiant.

12. Utilisation de la composition selon l'une quelconque des revendications 6 à 9 lors de la fabrication d'encres, de teintures, d'adhésifs ou de composants d'adhésifs, de laques, de plastisols, de masses de bouchage, comme plastifiants, en particulier dans des matériaux synthétiques ou des composants de matériaux synthétiques.

13. Utilisation de la composition selon l'une quelconque des revendications 6 à 9 comme solvant lors de la fabrication d'encres, de teintures, d'adhésifs ou de composants d'adhésifs, de laques, de plastisols, de masses de bouchage.

14. Utilisation de la composition selon l'une quelconque des revendications 6 à 9 comme composant d'huile lubrifiante.

15. Utilisation de la composition selon l'une quelconque des revendications 6 à 9 comme adjuvant lors de la transformation de métaux.
